# EUROPÄISCHE PATENTSCHRIFT

(11) **EP 1 463 688 B1**
(45) Veröffentlichungstag und Bekanntmachung des Hinweises auf die Patenterteilung: **13.07.2005**
(21) Anmeldenummer: 02791808.5
(22) Anmeldetag: 11.12.2002
(51) Int. Cl.: C03C 10/04

(54) **VERWENDUNG EINER ANTIMIKROBIELLEN GLASKERAMIK FÜR ZAHNPFLEGE, MUNDHYGIENE**
USE OF AN ANTIMICROBIAL GLASS CERAMIC FOR DENTAL CARE AND ORAL HYGIENE
UTILISATION D'UNE VITROCERAMIQUE ANTIMICROBIENNE POUR LES SOINS DENTAIRES ET L'HYGIENE BUCCALE

(30) Priorität: 12.12.2001 DE 10161074; 07.09.2002 DE 10241496
(43) Veröffentlichungstag der Anmeldung: 06.10.2004
(73) Patentinhaber: SCHOTT AG, 55122 Mainz (DE)
(72) Erfinder: ZIMMER, José, 55218 Ingelheim (DE); FECHNER, Jörg, Hinrich, 55118 Mainz (DE)
(74) Vertreter: Sawodny, Michael-Wolfgang, Dr. Dipl.-Phys.
(86) Internationale Anmeldenummer: PCT/EP2002/014042
(87) Internationale Veröffentlichungsnummer: WO 2003/050053

(56) Entgegenhaltungen:
- WO-A-93/17976
- WO-A-97/27148
- US-A- 5 120 340
- US-A- 5 735 942
- US-A- 5 981 412
- US-A- 6 086 374
- US-B1- 6 244 871

## Beschreibung

Gegenstand der Erfindung ist die Verwendung einer antimikrobiell, entzündungshemmend, remineralisierend, zahnsteinreduzierend wirkenden Glaskeramik bzw. eines antimikrobiell wirkenden Glaskeramikpulvers in der Zahnpflege. Ein mögliches Ausgangsglas für eine derartige Glaskeramik bzw. ein derartiges Glaskeramikpulver umfasst 30 - 75 Gew.-% SiO₂, 0 - 40 Gew.-% Na₂O, 0 - 40 Gew.-% CaO, 0 -20 Gew.-% P₂O₅.wobei die Summe Na₂O + K₂O + CaO < 70 Gew% und > 10 Gew.%

Gläser mit bioaktiver und teilweise auch antimikrobieller Wirkung werden bei LL. Hensch, J. Wilson, An Introduction to Bioceramics, World Scientific Publ. , 1993, als Bioglas beschrieben. Derartiges Bioglas zeichnet sich durch die Bildung von Hydroxylappatitschichten in wässrigen Medien aus. Schwermetallfreie Alkali-Erdalkali-Silicat-Gläser mit antimikrobiellen Eigenschaften werden in den Anmeldungen DE-A-199 32 238 und DE-A-199 32 239 beschrieben.

Aus der US 5,676,720 ist ein Glaspulver bekannt geworden, das 40-60 Gew.-% SiO₂, 5-30 Gew.& Na₂O, 10-35 Gew.-% CaO, 0-12 Gew.% P₂O₅ umfasst, wobei auch eine Glaskeramik, hergestellt aus einem Glas einer derartigen Zusammensetzung bekannt geworden ist. Allerdings ist in der US 5,676,720 keine Angabe über die Kristallphase gemacht.

US 5,981,412 beschreibt eine bioaktive Biokeramik für medizinische Anwendungen mit der kristallinen Phase Na₂O • 2CaO •3SiO₂. Die Kristallitgröße liegt bei 13 µm. Die Keramisierung erfolgt mit Temperschritten für Keimbildung und Kristallistion. Schwerpunkt liegt auf den mechanischen Eigenschaften wie z.B. K_{1c}. Der Kristallphasenanteil liegt zwischen 34 und 60 Vol.-%. Die US 5,981,412 beschreibt nur eine kristalline Phase bei der es sich um eine Hochtemperatur-Phase handelt und die nur unter den in dieser Schrift angegebenen speziellen Bedingungen entsteht. Eine Anwendung im Bereich der Zahnpflege ist nicht beschrieben.

Die Verwendung von bioaktiven Gläsern für Zahnpasta und Gels ist beispielsweise in der WO 97/27148 beschrieben. Aus der US 5,427,768 und der US 5,268,167 sind anorganische nichtmetallische Materialien bekannt, die Ca und Phosphor enthalten und durch entsprechende lonenabgaben zu einer Remineralisierung der Zähne führen.

Nachteilig an der Verwendung von der oben genannten Gläser im Bereich der Zahnpflege bzw. der Mundhygiene ist, dass Ionen nur unkontrolliert freigesetzt werden und diese Gläser in ihrer abrasiven Wirkung (Verwendung als Poliermittel) nicht einstellbar sind..

Es besteht daher die Aufgabe, Materialien für die Zahnpflege und/oder Mundhygiene anzugeben, die die Nachteile des Standes der Technik nicht aufweisen, insbesondere sollen die Materialien einen definiert einstellbaren abrasiven Effekt aufweisen. Andererseits sollen die Materialien eine antimikrobielle Wirkung aufweisen. Weiterhin sollen diese Materialien eine beschleunigte Reminerlisierung des Zahnes bewirken sowie die Bildung von Zahnstein vermindern und das Zahnfleisch durch die entzündungshemmende Wirkung stärken.

Die Erfinder haben nun herausgefunden, dass die Nachteile des Standes der Technik überwunden werden können, wenn die in dieser Erfindung beschriebenen Glaskeramiken oder Glaskeramikpulver im Bereich der Zahnpflege und/oder der Mundhygiene verwendet werden.
Glaskeramik Pulver umfassen in dieser Erfindung auch Glaskeramik Fasern, Kugeln,(Durchniesser < 100µm bevorzugt < 20µm) und Glaskeramik-Kügelchen (Durchmesser < 100µm, bevorzugt <20µm)
Die erfindungsgemäße Glaskeramik kann im Bereich der Zahnpflege und/oder der Mundhygiene als Poliermittel für Zähnde, Mittel gegen Zahnstein, Desensibilisierungsmittel für empfindliche Zähne, zur Verhinderung der Bildung von Säuren im Mundbereich und damit zur Verringerung der Kariesbildung sowie durch seine antimikrobielle Wirkung zur Vermeindung von Mundgeruch sowie insbesondere zur Remineralisierung der Zahne bzw. zum Aufbau einer Mineralschicht auf der Zahnoberfläche verwendet werden.

Besonders geeignet ist eine Glaskeramik bzw. ein Pulver aus einer derartigen Glaskeramik, die neben den antimikrobiellen Eigenschaften auch entzündungshemmende, remineralisrende, zahnsteinvermindernde, desensibilisierende Eigenschaften aufweist.

Bevorzugt sind Glaskeramiken, insbesondere Glaskeramikpulver, wobei das Ausgangsglas die nachfolgende Zusammensetzung in Gew.-% bezogen auf das Oxid aufweist:

| | |
|---|---|
| SiO₂ | 30 - 70 Gew.-% |
| Na₂O | 0 - 40 Gew.-% |
| K₂O | 0 - 40 Gew.-% |
| CaO | 5 - 40 Gew.-% |
| MgO | 0 - 40 Gew.-% |
| Al₂O₃ | 0 - 5 Gew.-% |
| P₂O₅ | 0 - 20 Gew.-% |
| B₂O₃ | 0 - 5 Gew.-% |
| ZnO | 0 - 10 Gew.-% |

und
0 bis 30 Gew.-% XF_{y}, wobei X Na, K, Mg, Ca sein kann und y = 1 oder y = 2 und insbesondere die Summe aus Na₂O + K₂O + CaO 5 - 70 Gew.-% beträgt.

Bei einer alternativen Ausgestaltung weist das Ausgangsglas

| | |
|---|---|
| SiO₂ | 30 - 60 Gew.-% |
| K₂O | 0 - 30 Gew.-% |
| Na₂O | 5 - 30 Gew.-% |
| CaO | 5 - 30 Gew.-% |
| P₂O₅ | 2 - 10 Gew.-% |
| ZnO | 0 - 10 Gew.-% |

und
0 bis 30 Gew.-% XFy, wobei X Na, K, Mg, Ca sein kann und y = 1 oder y = 2, in Gew.-% bezogen auf das Oxid auf.

In einer weiteren Ausgestaltung weist das Ausgangsglas nachfolgende Zusammensetzung auf:

| | |
|---|---|
| SiO₂ | 30 - 60 Gew.-% |
| K₂O | 5 - 30 Gew.-% |
| Na₂O | 0 - 30 Gew.-% |
| CaO | 5 - 30 Gew.-% |
| P₂O₅ | 2 - 10 Gew.-% |
| ZnO | 0 - 10 Gew.-% |

und
0 bis 30 Gew.% XF_{y}, wobei X Na, K, Mg, Ca sein kann und y = 1 oder y = 2, in Gew.-% bezogen auf Oxidbasis.

Besonders bevorzugt besteht die kristalline Hauptphase der aus den oben genannten Ausgangsgläsern erhaltenen Glaskeramik aus Alkali-Erdalkali-Silicaten und/oder Erdalkali-Silicaten und/oder Alkali-Silicaten.

Die erfindungsgemäße Glaskeramik bzw. das erfindungsgemäße Glaskeramikpulver zeichnet sich dadurch aus, dass sie gegenüber Bakterien, Pilzen sowie eine biozide, auf jeden Fall eine biostatische Wirkung aufweisen. Die Glaskeramik gemäß der Erfindung ist im Kontakt mit dem Menschen schleimhautverträglich und toxikologisch unbedenklich.

Bei Verwendung der erfindungsgemäßen Glaskeramik im Bereich der Zahnpflege und/oder der Mundhygiene ist die Maximalkonzentration an Schwermetallen beispielsweise für Pb < 20ppm, Cd < 5 ppm, As < 5 ppm, Sb < 10 ppm, Hg < 1 ppm, Ni < 10 ppm. n.
Ausnahmen können hierbei Farbgläser sein, die z.B. durch Dotierung mit farbgebenden Ionen oder Kolloiden erzeugt werden.
Sämtliche Angaben in der Anmeldung beziehen sich auf Gew.-% auf Oxidbasis.

Das unkeramisierte Ausgangsglas, das für die Herstellung der erfindungsgemäßen Glaskeramik verwandt wird, enthält SiO₂ als Netzwerkbildner zwischen 30 - 70 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität ebenfalls abnehmen und die Verarbeitungstemperatur wird deutlich erhöht, so dass sich die Heißformgebungseigenschaften verschlechtern. SiO₂ ist außerdem Bestandteil der bei der Keramisierung entstehenden kristallinen Phasen und muss, sofern hohe kristalline Phasenanteile durch die Keramisierung eingestellt werden sollen, in entsprechend hohen Konzentrationen im Glas enthalten sein.

Na₂O wird als Flussmittel beim Schmelzen des Glases eingesetzt. Bei Konzentrationen kleiner 5 % wird das Schmelzverhalten bei Abwesenheit weiterer Alkalioxide negativ beeinflusst. Natrium ist ein Bestandteil der sich bei der Keramisierung bildenden Phasen und muss, sofern hohe kristalline Phasenanteile durch die Keramisierung eingestellt werden sollen, in entsprechend hohen Konzentrationen im Glas enthalten sein.

K₂O wirkt als Flussmittel beim Schmelzen des Glases. Außerdem wird Kalium in wässrigen Systemen abgegeben. Liegen hohe Kaliumkonzentrationen im Glas vor, werden kaliumhaltige Phasen wie Kalium-Silicaten ebenfalls ausgeschieden. Die Summe der Alkalien sollte zur Gewährleistung der Schmelzbarkeit größer 5 Gew.-% sein. Der K₂O-Gehalt kann im Bereich 0-40 Gew-%, bevorzugt 0-25 Gew-%, besonders bevorzugt 0-10 Gew-% liegen.

Über den P₂O₅-Gehalt wird die chemische Beständigkeit des Glases und damit die Ionenabgabe in wässrigen Medien eingestellt. Der P₂O₅-Gehalt liegt zwischen 0 und 15 Gew.-%. Bei höheren P₂O₅-Werten wird die hydrolytische Beständigkeit der Glaskeramik zu gering.

Um die Schmelzbarkeit zu verbessern, kann das Glas bis zu 5 Gew.-% B₂O₃.

Die Menge an Al₂O₃ sollte kleiner 5 Gew.-% sein, um eine nicht zu hohe chemische Resistenz zu erreichen. Al₂O₃ wird genutzt, um die chemische Beständigkeit des Glases einzustellen.

Zur Verstärkung der antimikrobiellen, insbesondere der antibakteriellen Eigenschaften der Glaskeramik können antimikrobiell wirkende Ionen wie z. B. Ag, Au, I, Ce, Cu, Zn, Sn in Konzentrationen kleiner 5 Gew.-% enthalten sein.

Weiterhin können Ionen wie z. B. Ag, Cu, Au, Li zur Einstellung der Hochtemperaturleitfähigkeit der Schmelze und damit zur verbesserten Schmelzbarkeit mit Hochfrequenz-Schmelzverfahren als Zusätze enthalten sein. Die Konzentration dieser Ionen sollte kleiner 5 Gew.-% sein.

Farbgebende Ionen wie z. B. Fe, Cr, Co, V, Cu, Mn, Ag können einzeln oder kombiniert in einer Gesamtkonzentration kleiner 1 Gew.-% enthalten sein.

Zur Zahnhärtung kann das für die Glaskeramik verwendete Grundglas 0 - 30 Gew.-% XF_{y} enthalten, wobei X Na, K, Mg, Ca sein kann und y = 1 oder y = 2.

Üblicherweise wird die erfindungsgemäße Glaskeramik in Pulverform eingesetzt. Die Keramisierung des Ausgangsglases kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muss gegebenenfalls auch erneut gemahlen werden, um Agglomerate, die während des Keramisierungsschrittes entstanden sind, zu entfernen. Die Mahlung kann sowohl trocken oder auch in wässrigen und nicht-wässrigen Mahlmedien erfolgen.

Entscheidender Vorteil der Keramisierung in Pulverform ist eine sehr kleine Kristallitgröße bei trotzdem hohen Gesamtphasenanteilen. Außerdem wachsen die Kristallite an Oberflächendefekten, die beim Mahlen erzeugt werden, von der Oberfläche. Die tribologische Aktivierung der Oberfläche erhöht die Reaktionsfähigkeit der Pulver.

Durch Mahlen werden sehr viele Oberflächenkeime erzeugt, so dass gleichzeitig sehr viele Kristalle zu wachsen beginnen und damit eine extrem kleine Kristallitgröße bei trotzdem hohen kristallinen Phasenanteilen erreicht werden kann. Eine separate zusätzliche Temperbehandlung zur Keimbildung wie z.B. bei der US 5,981,412 beschrieben ist somit nicht erforderlich.

Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm. Als ganz besonders geeignet haben sich Partikelgrößen < 1 µm herausgestellt.

Mischungen verschiedener Glaspulver aus dem Zusammensetzungsbereich mit unterschiedlichen Zusammensetzungen und Korngrößen sind möglich, um bestimmte Effekte zu kombinieren.

Wird ein Block oder ein Ribbon eines Ausgangsglases keramisiert, so liegen die Kristallitgrößen im Bereich größer 10 µm, sofern kristalline Phasenanteile von größer 30 Vol.-% angestrebt werden. Die Kristallisation läuft sehr schnell ab. Die Keramisierungstemperaturen liegen zwischen 50° C und 400° C oberhalb Tg. Die Keramisierung kann hierbei auch in mehrstufigen thermischen Prozessen durchgeführt werden. Die Kristallisation ist primär oberflächengesteuert. Es wachsen nadelförmige Kristallite von der Oberfläche ins Glasinnere. Wenige Kristallite beginnen auch im Glasinneren zu wachsen. Sie sind sphärolitisch ausgeprägt. Bei der Keramisierung der Pulver entstehen wegen der hohen Oberflächen primär nadelförmige Kristalle.

Die Keramisierung des Ausgangsglases ist oberflächengesteuert. Werden die Ribbons bzw. Blöcke des Ausgangsglases vor der Keramisierung zu Pulvern gemahlen, so verschieben sich die Kristallisationstemperaturen zu deutlich niedrigeren Werten. Die Kristalle beginnen von den Oberflächen der Pulverpartikel ins Innere zu wachsen. Die Keramisierung kann so geführt werden, dass die Partikel nur eine äußere kristalline Schicht aufweisen und im Inneren amorph bleiben. Die Wahl der Partikelgröße bestimmt die mittlere Kristallitgröße. Durch eine Teilkristallisation der Partikel an der Oberfläche können besondere Effekte bei der Reaktion und lonenabgabe des teilkeramisierten Pulvers erreicht werden. Wird z.B. an der Oberfläche eine Phase generiert, die reaktiver ist als das Glas können Kurzzeit u. Langzeitverhalten der lonenabgabe kombiniert werden.
Die Kristallphasenanteile im Glas nach der Keramisierung sind größer als 5 Gew.-%. Je nach Zusammensetzung des Ausgangsglases können bis nahezu 100 Gew.-% kristalliner Phasenanteil erreicht werden.
Ein bevorzugter Bereich liegt bei einem Phasenanteil größer 10 Gew.-% sowie größer 30 Gew.-%. Noch bevorzugter ist der Bereich größer 50 Gew.-%.

Je nach Keramisierungstemperatur werden die keramisierten Pulver erneut aufgemahlen, um Agglomerationen, die während der Keramisierung entstanden sind, nochmals zu lösen.

Kristallhauptphasen sind Alkali-Erdalkali-Silicate und/oder Erdalkalisilicate, insbesondere NaCa-Silicate und Ca-Silicate, wobei diese Phasenanteile durch die Keramisierung beeinflusst werden können.

Weitere Kristallnebenphasen, die Silber und/oder Phosphor und/oder Silizium wie beispielsweise AgPO₃, SiP₂O₇, SiO₂ enthalten können, können je nach spezieller Zusammensetzung des Ausgangsglases ebenfalls auftreten.

Phosphorhaltige Glaskeramiken aus diesem Zusammensetzungsbereich können in wässrigen Medien bioaktiv sein, d.h. sie bilden in wässrigen Systemen an ihrer Oberfläche und auch an Fremdoberflächen eine Hydroxylapatit-Schicht. Daher sind solche Pulver besonders geeignet, um als Biomaterialien oder in Anwendung, in denen Remineralisierungsprozesse eine wichtige Rolle spielen, d.h. z.B. im Bereich der Zahnpflege verwendet zu werden.

Durch die Phasen und Phasenanteile wird die chemische Reaktivität bzw. die lonenabgabe beeinflusst. Chemische Reaktivität und lonenabgabe können somit gesteuert werden, so dass hierüber die Hauptverträglichkeit, der pH-Wert sowie antimikrobielle und entzündungshemmende Wirkung eingestellt werden kann.

Die kristallinen Phasen zeigen eine deutlich andere chemische Beständigkeit als die Glasphase. Die chemische Beständigkeit kann sowohl erhöht als auch emiedrigt sein. Neben den chemischen Eigenschaften werden entsprechend den Kristallhauptphaseneigenschaften auch die mechanischen, abrasiven und optischen Eigenschaften modifiziert.

Bei relativ niedrigen Keramisierungstemperaturen < 700°C bei Ribbons werden zunächst ein bis zwei Na-Ca-Silikate gebildet. Bevorzugt sind dies (Na₂CaSi₃O₈ / Na₂CaSiO₄) / Na₂Ca₂(SiO₃)₃. Bei Temperaturen größer 700°C findet eine Umkristallisation statt.

Die entstehenden kristallinen Phasen zeigen teilweise eine wesentlich höhere Wasserlöslichkeit als die Glasphase. Durch das gezielte Einstellen der Phasenanteile kann somit die lonenabgabe der Pulver sowie der pH-Wert in wässriger Lösung und damit auch die biologische Wirkung beeinflusst werden.

Beim Auflösen der kristallinen Phase in Wasser oder wässriger Lösung bleiben wabenförmige bzw. poröse Oberflächenstrukturen zurück, die insbesondere optische Eigenschaften wie Transmission, Reflektion und Lichtstreuung der Pulver in Formulierungen beeinflussen. Beim Anlösen in wässrigen Systemen wird ebenfalls eine Bildung von Nanopartikeln beobachtet.

Die erfindungsgemäßen Glaskeramikpulver können in einer Vielzahl von Formulierungen für die Zahnpflege bzw. die Mundhygiene eingesetzt werden. Nur beispielsweise seien hier Zahnpasten erwähnt. Durch Auflösen der kristallinen Phasen entstehen Nanopartikel. Diese Nanopartikel können u.a. Tubulinkanäle im Zahn verschließen und somit eine desensibilisierende Wirkung auf empfindliche Zähne besitzen. Die Formulierungen für die Zahnpflege bzw. die Mundhygiene, in denen die erfindungsgemäße Glaskeramik verwendet wird, können wasserfrei sein oder auch Wasser enthalten. Besonders bevorzugt ist es, wenn die Partikelgröße des Glaspulvers kleiner 10 µm ist. Bei Verwendung der erfindungsgemäßen Glaskeramik bzw. Glaskeramikpulver in Formulierungen für die Mundhygiene und Mundpflege wird gegenüber dem Einsatz von reinem Glaspulver aufgrund der Keramisierung erreicht, dass die Pulver und damit die Formulierung in ihrer Reaktivität definiert eingestellt werden können, insbesondere auch reaktiver sein können als die entsprechenden Gläser. Wird anstelle eines Glaspulvers ein Glaskeramikpulver eingesetzt, so sind die Bildung der Hydroxylapatit-Schicht und die lonenabgabe erheblich beschleunigt. Gegenüber im Bereich der Zahnpflege verwendeten Glaspulver ergeben sich hieraus erhebliche Vorteile, da nur eine relativ kurze Einwirkzeit benötigt wird. In Formulierungen für die Zahnpflege kann das erfindungsgemäße Glaskeramikpulver wirksam Zahnfleischbluten und Parodontose vorbeugen sowie Mundgeruch unterdrücken und dem Weißen der Zähne dienen. Als Formulierungen aus dem Bereich der Zahnpflege seien Mundwasser, Zahnpasta oder Zahnseide beispielhaft genannt. Verwendet man die erfindungsgemäßen Glaskeramiken in derartigen Formulierungen, so kann auf Konservierungsmittel bzw. andere antimikrobiell wirkende Stoffe, wie beispielsweise Triclosan, verzichtet werden. Außerdem wird durch die Abgabe von Ca und P₂O₅ die Remineralisierung der Zahnoberflächen beschleunigt. Durch die entzündungshemmende Wirkung der Glaskeramiken bzw. der Glaskeramikpulver wird außerdem Zahnfleischentzündungen vorgebeugt und Zahnfleischbluten kann wirksam behandelt werden. In Formulierungen für die Zahnpflege bzw. die Mundhygiene liegt die Konzentration des Glaskeramikpulvers bevorzugt in Bereichen 0,1 bis 10 Gew.-%, besonders bevorzugt 1 bis 10 Gew-%, insbesondere bvorzugt 0,1 - 5 Gew-% bezogen auf die Gesamtzusammensetzung.

In den Formulierungen für die Zahnpflege kann das Glaspulver auch als Schleifmittel dienen und zu einer Desensibilisierung der Zähne.

Die bessere und schnelle Bildung der Hydroxylapatit-Schichten gegenüber den bislang bekannten und in Formulierungen für die Zahnpflege und Mundhygiene eingesetzten Glaspulvern liegt darin, dass ein bereits kristallisiertes Material vorliegt. Es können sich dann kristalline Subeinheiten auf der Zahnoberfläche bzw. in den Tubulinkanälen ablagern. Durch die Auflösung der Kristalle schon in der Formulierung bzw. während der Applikation bilden sich Nanopartikel, die sich teilweise auflösen, bzw. ebenfalls auf der Zahnoberfläche ablagern bzw. in die Tubulin-Kanäle wandern. Die Nanopartikel sind besonders dafür geeignet, sich auf der Zahnoberfläche abzulagem und dort eine Mineralschicht aufzubauen. Dies ist insbesondere der Fall, wenn das Glaskeramikpulver Fluoride enthält. Der Vorteil gegenüber Glaspulver liegt darin, das sich diese kristallinen Nanopartikel in Unebenheiten auf der Zahnoberfläche festsetzen und dort auch nach dem Spülen verbleiben und somit eine Langzeithärtung des Zahnes durch freigesetztes Fluorid bzw. Calzium und Phosphor erfolgt.

Insbesondere kann aufgrund der Bildung von Hydroxylapatit und Nanopartikeln erreicht werden, dass sich tubulare Kanäle schließen. Durch Verwendung der erfindungsgemäßen Glaskeramik bzw. des Glaskeramikpulvers in Mitteln für die Zahnpflege oder Mundhygiene können Kalium-lonen oder Calcium-Ionen freigesetzt werden, welche einen zahnschmerzinhibierenden Effekt besitzen und durch exponierte Dentinkanäle zu Zahnnerven oder -neuronen gelangen können und diese desensibilisieren. Dadurch wird ein schmerzfreieres Zähneputzen ermöglicht und die Empfindlichkeit der Zähne gegnüber Heiß-Kalt Wechseln verringert (Desensibilisierung der Zähne bzw. der Zahnnerven/Zahnwurzel).

Des weiteren können aus der Glaskeramik bzw. dem Glaskeramikpulver Fluorid-lonen freigesetzt werden, welche den Zahn härten. Aufgrund der Freisetzung von Polyphosphaten aus der Glaskeramik bzw. dem Glaskeramikpulver wird die Bildung von Zahnstein verringert sowie vorhandener Zahnstein abgebaut. Eine weitere Komponente, die gegen Zahnstein wirksam ist, sind ZinkIonen. Diese können ebenfalls Bestandteil des Glaskeramikpulvers sein und in wässriger Lösung aus dieser freigesetzt werden.

Eine andere erwünschte Komponente, beispielsweise in Zahnpasten oder Zahncremes, -gelen, -putzmitteln ist ein synthetisches anionisches polymeres Polycarboxylat (SAPP), das als Stabilisator für das gegen Zahnstein wirkende Polyphosphat, welches aus der Glaskeramik freigesetzt wird, wirkt und dazu beiträgt, den Zugang von schmerzenden oder schmerzverursachenden Materialien wie Zucker zu den Zahnnerven zu blockieren.

Anstelle der erwähnten polymeren Polycarboxylate kann man auch andere SAPP-Typen verwenden, zumindest vorzugsweise nur teilweise, zum Beispiel Polysulfonate, Polysulfonate und Polyphosphonate, zum Beispiel typischerweise gewöhnlich bis zur Hälfte des SAPP-Gehalts. Die verschiedenen Polymere dieser Typen können hergestellt werden durch Umsetzen einer ethylenisch ungesättigten organischen Säure wie Malein-, Croton-, Sorbin-, α-Chlorsorbin-, Zimt-, Mucon-, Itacon-, Citracon-, Mesacon-, Glutacon-, Aconit-, Angelika-, Umbell- oder Furmarsäure(n) oder Anhydrid(e) mit einer geeigneten polymerisierten ethylenisch ungesättigten Carbon-, Sulfon-, Schwefel- oder Phosphonsäure, die eine aktivierte olefinische Kohlenstoff-Kohlenstoffdoppelbindung und mindestens eine Carboxyl-, Sulfon-, Schwefelsäure- oder Phosphonsäuregruppe enthält. Andere olefinische Monomere, die mit den beschriebenen Säuren oder Anhydriden copolymerisierbar sind, umfassen Vinylacetat.Vinylchlorid, Dimethylmaleat und ähnliche ungesättigte Monomere und die hergestellten Copolymere werden eine ausreichende Menge an sauren oder neutralisierten oder neutralisierbaren sauren Gruppen enthalten, um sie in Wasser löslich und quellbar zu machen. Einige derartige Polycarboxylat-Copolymere sind in US-A-41 38 477 und 41 83 914 beschrieben.

Die aus der Glaskeramik in wässriger Lösung, beispielsweise dem Mundspeicher, freigesetzten Fluorid-lonen tragen dazu bei, die ebenfalls aus der Glaskeramik freigesetzten Phosphate gegen enzymatischen Angriff zu stabilisieren, während sie den Zusammensetzungen auch ihre zahnhärtenden und Antikarieseigenschaften vermitteln. Zusätzlich zu den aus der Glaskeramik bzw. dem Glaskeramikpulver freigesetzten Fluorid-lonen können der Formulierung auch andere Lieferanten von Fluorid-lonen zugesetzt werden, wie zum Beispiel wasserlösl. Alkalimetallfluoride, wie Na- und K-Fluoride, Kupferfluoride, wie Cuprofluorid, Zinnfluoride wie Stannofluorid, Ammoniumfluorsilikat, Natriumund Ammoniumfluorzirkonate, Natrium- und Kaliummonofluorphosphate, Aluminiumfluorphosphate (Mono-, Di- und Tri-) und fluoriertes Natriumcalciumpyrophosphat.
Der oral verträgliche Träger oder Grundstoff für die erfindungsgemäße Zusammensetzung wird, wenn derartige Zusammensetzungen Zahnpasten sind, was bevorzugt ist, normalerweise Wasser, Feuchthaltemittel, körpergebende Substanz, Surfactant (Tensid) oder Detergens (synthetisches Tensid) und Poliermittel umfassen. Das angewandte Wasser kann jedes trinkbare Wasser sein, vorzugsweise soll es jedoch eine geringere Härte als 200 ppm als Calciumcarbonat, und besonders bevorzugt weniger als 100 ppm Härte haben. Am meisten bevorzugt ist entmineralisiertes und bestrahltes Wasser. Wasser und Feuchthaltemittel machen den flüssigen Teil der Zahnpasta aus. Die Feuchthaltemittelkomponente der Zahnpasta umfasst vorzugsweise eine Mischung mehrerer Feuchthaltemittel wie Glycerin, Sorbit und Polyethylenglykol, das am meisten bevorzugt ist, jedoch können andere Mischungen von Feuchthaltemitteln und einzelne Feuchthaltemittel ebenfalls verwendet werden. Zu anderen Feuchthaltemitteln, die brauchbar sind, gehören Propylenglykol und Polypropylenglykole. Ein normaler Molekulargewichtsbereich für die Polyethylenglykol-Feuchthaltemittel ist 200 bis 1000, vorzugsweise 400 bis 600 oder 800, zum Beispiel etwa 600.

Körpergebende Substanz, Geliermittel oder Verdicker des Zahnpastagrundstoffs kann jedes derartige Agens sein, doch finden sich die meisten von diesen in den Klassen natürlicher und synthetischer Gummen und Kolloide. Von diesen können Carrageean (Irish Moos), Kanthangummi und Natriumcarboxymethylcellulose genannt werden, die bevorzugt sind, und ebenso Gummitragacanth, Stärke, Polyvinylpyrrolidon, Hydroxyethylpropylcellulose, Hydroxybutylmethylcellulose, Hydroxypropylmethylcellulose und Hydroxyethylcellulose (die als Natrosol® erhältlich ist). Anorganische Verdicker, wie kolloidale Kieselsäure, z.B. Syloid®244, und synthetischer Hectorit, zum Beispiel Laponite®, der von Laporte Industries, Ltd. verkauft wird, können ebenfalls verwendet werden und Mischungen dieser Verdickungsmittel sind ebenfalls brauchbar.

Die oberflächenaktiven Substanzen oder Tenside sind normalerweise ein wasserlösliches synthetisches Tensid, das zum Reinigen der Zähne (und des Zahnfleischs) geeignet ist und dazu beiträgt, die gegen Zahnstein wirkenden und desensibilisierenden Komponenten der Zusammensetzung mit den Zahnflächen in Kontakt zu bringen und in Dentin und Pulpa, sofern exponiert, einzudringen.

Derartige synthetische Tenside haben wertvolle Schäumeigenschaften und tragen auch dazu bei, eine gleichförmige Zahnpasta zu erzeugen, in welcher die aktiven Komponenten gleichmäßig verteilt sind, so dass jede einer reichlich belegten Zahnbürste entsprechende Menge an Zahnpasta (toothbrushful of toothpaste) wirksame Menge dieser Materialien enthalten wird. Das organische, oberflächenaktive Material ist vorzugsweise anionisch, nichtionisch oder ampholytisch, und am meisten bevorzugt anionisch. Geeignete Beispiele von Aniontensiden sind Höheralkylsulfate wie Kaliumlaurylsulfat, Monoglyceridmonosulfate von höheren Fettsäuren wie das Kaliumsalz des monosulfatierten Monoglycerids von hydrierten Kokosnussölfettsäuren. Alkylarylsulfonate wie Kaliumdodecylbenzolsulfonat, höher-Fettsulfoacetate, Ester höherer Fettsäuren von 1,2-Dihydroxypropansulfonat, und die im wesentlichen gesättigten höheraliphatischen Acylamide von niederaliphatischen Aminocarbonsäureverbindungen, wie jene mit 12 bis 16 Kohlenstoffatomen in der Fettsäure, Alkyl- oder Acylresten und dergleichen. Beispiele der zuletzt erwähnten Amide sind N-Lauroylsarcosin und die Kaliumsalze von N-Lauroyl-, N-Myristoyl- oder N-Palmitoylsarcosin, die im wesentlichen frei sein sollen von Seife oder ähnlichem höherem Fettsäurematerial. Obgleich es bevorzugt ist, Kaliumtensid anzuwenden, sind diese häufig im Handel nicht erhältlich; in diesen Fällen kann man Natriumsalze anwenden (und manchmal mögen diese sogar in den beschriebenen Zahnpasten bevorzugt sein).

Beispiele für wasserlösliche Niotenside sind Kondensationsprodukte von Ethylenoxid mit verschiedenen wasserstoffhaltigen Verbindungen, die mit diesen reaktiv sind und lange hydrophobe Ketten aufweisen (zum Beispiel aliphatische Ketten von etwa 12 bis 20 Kohlenstoffatomen), wobei diese Kondensationsprodukte ("Ethoxamere") hydrophile Polyoxyethylenanteile enthalten, zum Beispiel Kondensationsprodukte von Poly (Ethlenoxid) mit Fettsäuren, Fettalkoholen, Fettamiden und anderen Fettresten, und mit Propylenoxid und Polypropylenoxiden (zum Beispiel (Pluronic®-Materialien). Von den erwähnten synthetischen Tensiden sind die Sulfate höherer Fettalkohole bevorzugt (in derartigen und in den anderen erwähnten synthetischen Tensiden und überall in dieser Beschreibung identifiziert das Wort "höher", wenn es zur Bezeichnung von Alkylgruppen, Fettsäuren usw. verwendet wird, solche, die 10 bis 20 Kohlenstoffatome, vorzugsweise 12 bis 18, vorzugsweise in linearer Anordnung, enthalten).

Die hier beschriebenen Glaskeramiken bzw. Glaskeramikpulver besitzen neben den oben beschriebenen Eigenschaften auch die Eigenschaft, als Poliermittel oder Schleifmittel in einer Zahnpasta zu fungieren, wobei jedoch nicht beabsichtigt ist, dass sie Zahnmaterial entfernen, sondern lediglich Ablagerungen von den Zähnen und diese polieren.

Verschiedene andere Komponenten von Zahnpasten können als zusätzliche aktive Materialien oder Hilfsstoffe in Betracht gezogen werden. Zu dieser Gruppe gehören: andere gegen Zahnstein oder Calculus wirkende Verbindungen wie AHP, PPTA, PBTA und EHDP, Zinkverbindungen wie Zinkchlorid, Zinkacetat und Zinkoxid, Sanquinariaextrat: antibakterielle Substanzen wie Triclosan; Puffer zum Steuern des pH-Wertes: Bleichmittel und Zahnweißungsmittel wie Perverbindungen: Schutzstoffe; Süßstoffe wie Kalium(oder Naterium)saccarin oder Cyclamat. Acesulfam-K, Sucralose und Aspartam; Aromastoffe wie Minze (Pfefferminz und Grüne Minze) und Menthol; und Farbstoffe und Pigmente wie Chlorphyll und Titanoxid. Derartige wasserlösliche aktive und Hilfsstoffe der Zahnpasten oder anderen Mundpflegemitteln der Erfindung sind, falls sie in löslicher Salzform vorliegen, vorzugsweise Kaliumsalze, da es scheint, dass Kaliumkationen in diesen Zusammensetzungen die Desensibilisierung der Zahnnerven durch das zahnschmerzinhibierende Kaliumsalz, zum Beispiel Kaliumnitrat und Kaliumcitrat, erhöhen.

Obgleich es bevorzugt ist, dass die Mundpflegemittel der Erfindung Zahnpasten oder gelförmige Zahnputzmittel (einschließlich gestreiften Zahnputzmitteln) sind, die auf die Zähne gebürstet werden, um sie zu reinigen und Zahnsteinbildung auf ihnen zu verhindern, können auch andere Formen von Mundpflegemitteln durch den Einbau der hier detailliert beschriebenen zahnsteinverhindernden und desensibilisierenden Bestandteile verbessert werden. Da die mechanischen Kräfte des Bürstens als solche irritierend sein können und irritierende Chemikalien aus Nahrungsmitteln, Zahnsteinablagerungen, Kandis und sogar von anderen Zahnpflegemitteln in das Dentin und die Pulpa von empfindlichen Zähnen pressen können, können Zahnpulver, Lotionen und flüssige Zahnputzmittel ebenfalls durch Einbau der erwähnten gegen Zahnstein wirkenden und desensibilisierenden Komponenten signifikant verbessert werden, obgleich die Erfindung die größte Anwendung in Zahnpasten findet. Andere Präparate zur Mundpflege, die nicht auf die Zähne gebürstet werden sollen, können ebenfalls die beschriebenen, gegen Zahnstein wirkenden und desensibilisierenden Komponenten enthalten und zu diesen Produkten gehören Mundspülmittel, antiseptische Lösungen, Kaugummi, Zahnbehandlungsmittel wie Plaque lokalisierende Lösungen und sogar Zahnseide und Zahnband. In diesen Präparaten, die keine Zahnpasta und kein gelförmiges Zahnpflegemittel sind, sind die Anteile an gegen Zahnstein wirkendem Pyrophosphat , bevorzugt aus dem Glaskeramikpulver freigesetzt oder verwandter Verbindung eine gegen Zahnstein wirkende Menge und die an desensibilisierenden Kaliumverbindungen, bevorzugt aus dem Glaskeramikpulver freigesetzt, aber auch der Formulierung zugesetzte Verbindungen wie z.b. Kaliumnitrat oder Kaliumcitrat wird eine desensibilisierende Menge sein. Die Mundspülmittel enthalten normalerweise Wasser, Alkohol, Feuchthaltemittel wie Glycerin, Sorbit und/oder Polyethlenglykol, Aromastoff und Süßstoff (nicht Zucker) zusätzlich zu den erwähnten Aktivkomponenten und die Zahnpulver neben dem u.a. auch als Poliermittel wirkenden Glaskeramikpulver auch weitere Poliermittel wie Zeodent, Syloid, Santocel oder Calciumcarbonat enthalten. Die Lotionen können einen Träger oder Grundstoff aus einem Gummi- oder polymeren Bindemittel enthalten wie Carrageenan oder Alginat (vorzugsweise Kaliummalginat) mit Glaskeramikpulver alsfüllstoff enthalten sein. Es können auch Füllstoffe wie Calciumcarbonat oder feinteiliger Kieselsäure (in Mikrongröße) verwendet werden. Kaugummis können ein natürliches oder synthetisches Elastomer oder Kautschuk als Grundstoff enthalten. In den anderen erwähnten Präparaten kann die gewöhnliche Produktformulierung das beschriebene gegen Zahnstein wirkende Argens und erfindungsgemäße desensibilisierende Agens ebenfalls enthalten oder diese können darauf abgelagert werden, wie beispielsweise auf Zahnseide und -band, und vorzugsweise sind dies Kaliumhaltige Glaskeramikpulver, die Kalium-Ionen freisetzen.

Zur Herstellung von Zahnpasten ist ein besonderes Verfahren bevorzugt, da es hervorragende Zahnpasten ergibt, die den gewünschten pH-Wert und Viskosität aufweisen, und in welchen die Aktivkomponenten verbesserte Stabilitäten haben. Bei diesem Verfahren werden die Glycerin- und Polyethylenglykolkomponenten des Feuchthaltemittels zuerst miteinander in einem üblichen Mischer vermischt und dann werden die Verdicker, das Copolymer, das Glaskeramikpulver sowie eventuell zusätzlich enthaltenes Alkalimetallfluorid und Kaliumpyrophosphat in dem Feuchthaltemittelgemisch unter Mischen dispergiert, und dieses Mischen wird fortgesetzt, bis die Mischung ein Brei oder eine Aufschlämmung von glattem Aussehen wird, wonach man der glatten Aufschlämmung den Sorbit zumischt und Wasser zugibt und die desensibilisierende(n) Substanze(n) mit der verdünnten Aufschlämmung vermischt. Alle diese Mischvorgänge erfolgen bei Zimmertemperatur in dem Bereich von 20 bis 30°C. Dann kann die erzeugte Gelphase auf eine Temperatur in dem Bereich von 55 bis 75°C erwärmt werden, wobei gemischt wird, und das Mischen wird 10 bis 30 Minuten fortgesetzt, nachdem die erhöhte Temperatur in dem gegebenen Bereich erreicht worden ist. Wenn das Copolymer anfangs in sauerer Form vorliegt, wird es dann dem basisch wirkenden Glaskeramikpulver bzw. evl. weitere Zusatz von Alkalimetallhydroxid, vorzugsweise Kaliumhydroxid, auf einen pH-Wert in dem Bereich von 6 bis 8, vorzugsweise 7, unter Vermischen neutralisiert bzw. schwach alkalisch eingestellt und dieses Vermischen wird während weiterer 10 bis 30 Minuten nach Vollendung der Zugabe des Alkalihydroxids fortgesetzt. Die erhaltene Gelphase wird dann, wenn sie erwärmt ist, auf eine Temperatur in dem Bereich von 35 bis 45°C abgekühlt, wonach weiteres Glaskeramikpulver und evl. eingesetzte kieselsäurehaltige Poliermittel mit der Gelphase vermischt werden und das Vermischen weitere 10 bis 30 Minuten unter Vakuum in dem Bereich von 5 bis 100 mm Quecksilber, vorzugsweise 5 bis 50 mm Quecksilber, fortgesetzt wird, was zur Bildung einer Paste oder eines Gels führt. Die letzte Stufe des Verfahrens (abgesehen von Zugaben von Pigment, Aromastoff, Süßstoff oder anderen Hilfsstoffen) ist das Vermischen von Tensid, vorzugsweise Aniontensid, mit der Paste oder dem Gel, wonach man weitere 3 bis 10 Minuten unter einem Vakuum von 5 bis 50 mm Hg mischt. Das erhaltene Produkt ist eine beständige, gegen Zahnstein wirkende, desensibilisierende Zahnpasta, die eine Viskosität ähnlich der von normalen Zahnpasten aufweist, etwa 100 000 bis 500 000 Centipoise, einen pH in dem Bereich von 7 bis 11, vorzugsweise 7 bis 10, zum Beispiel 8, von zufriedenstellendem Geschmack ist (insbesondere bei Anwesenheit eines Minze/Mentholaromastoffs) und nicht übermäßig salzig ist. Bei der obigen Beschreibung des Herstellungsverfahrens wurde eine Feuchthaltemischung angewandt und Zugaben von Geschmacksstoff, Süßstoff und Pigment wurden nicht erwähnt. Wenn eines oder mehrere der beschriebenen Feuchthaltemittel oder irgendwelche andere optimale Bestandteile in der Formulierung nicht anwesend sind, können die oben erwähnten Zugabestufen, in welchen diese Bestandteile eingebracht werden, weggelassen werden. Auch können Süßstoff und Pigment mit dem Verdicker, Copolymer, Fluorid und Glaskeramikpulver und evl. engesetztem Polyphosphat dem Glycerin/Polyethlenglykolgemisch zugegeben werden und der Aromastoff kann mit dem Tensid gegen Ende des Verfahrens zugegeben werden.

Die erfindungsgemäßen Zahnpasten können mittels anderer Verfahren als dem oben beschriebenen hergestellt werden, doch wurde gefunden, dass das beschriebene Verfahren bessere Zahnpasten ergibt, weshalb es bevorzugt ist. Was die anderen Ausbildungsweisen der Erfindung betrifft, so können gelförmige Zahnputzmittel im wesentlichen in der gleichen Weise hergestellt werden, mit normalen Einstellungen der Formelbestandteile und Mengen, die dem Fachmann bekannt sind. Die Herstellung von Zahnpulver ist nur eine Sache des Vermischens der verschiedenen aktiven Bestandteile, und zur Herstellung von Mundwässem oder anderen flüssigen Präparaten können die hauptsächlichen Aktivkomponenten in einem geeigneten flüssigen Medium, gewöhnlich einem wässrig-alkoholischen Medium gelöst oder dispergiert werden, und Polymere, gummiartige und unlösliche Materialien werden normalerweise weggelassen, obgleich das SAPP anwesend sein kann. Andere Arten von Mundpflegemitteln oder Präparaten können nach geeigneten bekannten Verfahren hergestellt werden, mit geeigneten Zugaben der üblichen aktiven Komponenten und den geeigneten üblichen Ergänzungs- und Hilfsstoffen während des Herstellungsverfahrens.

Eine besonders bevorzugte Verwendung des beschriebenen Glaskeramikpulvers ist der Einsatz in wasserfreien Produkten der Mundhygienen bzw. Zahnpflege wie z.B. wasserfreie Zahncreme.
Die folgenden Beispiele und Figuren sollen die Erfindung erläutern und nicht den Schutzumfang beschränken. Wenn nicht anders angegeben, beziehen sich alle Teile und Prozentsätze auf das Gewicht und alle Temperaturen auf °C.

Es zeigen:
- Fig. 1: Röntgenbeugungsdiagramm eines in Pulverform kristallisierten Ausgangsglases mit einer Zusammensetzung gemäß Ausführungsbeispiel 1, getempert für 5 h bei 650°C.
- Fig. 2: Röntgenbeugungsdiagramm eines in Pulverform kristallisierten Ausgangsglases, getempert für 5 h bei 590°C.
- Fig. 3: Röntgenbeugungsdiagramm eines in Pulverform kristallisierten Ausgangsglases, getempert für 5 h bei 560°C.
- Fig. 4: DTA-Analyse eines als Glasblock keramisierten Ausgangsglases gemäß Ausführungsbeispiel 1.
- Fig. 5: DTA-Analyse eines in Pulverform keramisierten Ausgangsglases gemäß Ausführungsbeispiel 1.
- Fig. 6: Röntgenbeugungsdiagramm von keramisierten Ribbons bei unter schiedlichen Temperaturen.
- Fig. 7: Hochtemperaturröntgendiagramme an Glaspulvern mit einer Partikelgröße von 4 µm in Abhängigkeit von der Temperatur für Glaskeramiken mit einem Ausgangsglas gemäß Ausführungsbeispiel 7
- Fig. 8: Röntgenbeugungsdiagramm eines kristallisierten Ausgangsglases mit einer Zusammensetzung gemäß Ausführungsbeispiel 8, getempert für vier Stunden bei 560°C
- Fig. 9: Röntgenbeugungsdiagramm eines kristallisierten Ausgangsglases mit einer Zusammensetzung gemäß Ausführungsbeispiel 8, getempert für vier Stunden bei 700°C
- Fig. 10: Röntgenbeugungsdiagramm eines kristallisierten Ausgangsglases mit einer Zusammensetzung gemäß Ausführungsbeispiel 8, getempert für vier Stunden bei 900°C
- Fig. 11: Röntgenbeugungsdiagramm eines kristallisierten Ausgangsglases mit einer Zusammensetzung gemäß Ausführungsbeispiel 9, getempert für vier Stunden bei 560°C
- Fig. 12: Röntgenbeugungsdiagramm eines kristallisierten Ausgangsglases mit einer Zusammensetzung gemäß Ausführungsbeispiel 9, getempert für vier Stunden bei 700°C
- Fig. 13: Röntgenbeugungsdiagramm eines kristallisierten Ausgangsglases mit einer Zusammensetzung gemäß Ausführungsbeispiel 9, getempert für vier Stunden bei 900°C
- Fig. 14: DTA-Analyse eines als Glasblock keramisierten Ausgangsglases gemäß Ausführungsbeispielen 8 und 9
- Fig. 15: Normierte Basenstärke für eine Glaskeramik, keramisiert bei verschiedenen Temperaturen, ausgehend von einem Ausgangsglas mit einer Zusammensetzung gemäß Ausführungsbeispiel 1
- Fig. 16: Normierte Leitfähigkeit für Glaskeramiken, keramisiert bei verschiedenen Temperaturen ausgehend von einem Ausgangsglas mit einer Zusammensetzung gemäß Ausführungsbeispiel 1
- Fig. 17: REM-Aufnahme eines unbehandelten Zahnes.
- Fig. 18: REM-Aufnahme eines mit 37%-iger Phosphorsäure H₃PO₄ für 60 sec. angelösten Zahnes.
- Fig. 19: REM-Aufnahme eines Zahnes nach einer Behandlung für 24 h mit einer 10% Gew-%igen wässrigernSuspension eines Glaskeramikpulver mit einem Ausgangsglas gemäß Ausführungsbeispiel 1, wobei das Pulver bei 900°C für 2 Stunden keramisiert wurde. Die Partikelgröße beträgt d₅₀=4µm. Der Maßstab der Aufnahme beträgt 200µm
- Fig. 20: REM-Aufnahme eines Zahnes, angeätzt mit 37 Gew-%iger H₃ PO₄ für 60 sec. Und anschließend 24 h Behandlung mit einer 10 Gew-% gen wässrigen Suspension eines Glaskeramikpulver ausgehend von einem Ausgangsglas gemäß Ausführungsbeispiel 1, die durch Keramisierung bei 900°C und Tempern für 2 Stunden erhalten wurde. Die Partikelgröße beträgt d₅₀=4µm.
- Fig. 21: EDX-Spektrum an der Stelle D in Figur 12
- Fig. 22: unbehandelter Zahn eines Erwachsenen
- Fig. 23: Zahn mit 60%iger H₃ PO₄ (60 sec) angeätzt
- Fig. 24: Zahn mit 60%iger H₃ PO₄ (60 sec) angeätzt, anschließend 24 h mit
5%-iger Suspension Ausf.1 beahndelt
- Fig.25: REM-Aufnahme der Oberflächenkristalle an der Oberfläche einer Glaskeramik, die durch Tempern eines Ausgangsglases gemäß Ausführungsbeispiel 1 bei 660°C für 4 h erhalten wurde
- Fig.26: REM-Aufnahme eines Schnittes durch eine Glaskeramik, die durch Bulkkristallisation durch Tempern bei T=660°C für 4 h erhalten wurde.
- Fig.27: Oberfläche eines Glaskeramikribbons keramisiert bei 700°C, anschließend mit Wasser für 15 Minuten behandelt
- Fig.28A-B: Oberfläche eines Glaskeramikpulvers, keramisiert bei 700°C, anschließend 24 h in Wasser behandelt.
- Fig.29A-B: Oberfläche eines Glaskeramikpulvers, keramisiert bei 900°C, anschließend 24 h in Wasser behandelt.

Zunächst soll die Herstellung der Glaskeramik bzw. des Glaskeramikpulvers ausgehend vom Ausgangsglas beschrieben werden.

Aus den Rohstoffen wurde ein Glas erschmolzen. Die Schmelze wurde in Platintiegeln bei 1550°C durchgeführt. Die Schmelze wurde anschließend zu Ribbons geformt. Diese Ribbons wurden mittels Trockenmahlung zu Pulver mit einer Partikelgröße d50 = 4 µm weiterverarbeitet.

In Tabelle 1 sind die Zusammensetzungen der Ausgangsgläser in Gew.-% angegeben.

**Tabelle 1:**

| Zusammensetzungen (Synthesewerte) [Gew.-%] | | | | | | | | | | | |
|---|---|---|---|---|---|---|---|---|---|---|---|
| Beispiel | 1 | 2 | 3 | 4 | 5 | 6 | 7 | 8 | 9 | 10 | 11 |
| SiO₂ | 46,0 | 35 | 46 | 50 | 40 | 59 | 45 | 44,9 | 35 | 45 | 65 |
| Al₂O₃ | 0 | 0 | 0 | 0 | 0 | 0 | 1 | 0 | 0 | 0 | 0 |
| CaO | 25,0 | 29 | 20 | 10 | 25 | 20 | 25 | 24,5 | 29,0 | 23,5 | 10,0 |
| MgO | 0 | 0 | 5 | 0 | 0 | 0 | 0 | 0 | 0 | 0 | 0 |
| Na₂O | 25,0 | 30 | 20 | 25 | 25 | 20 | 24 | 24,5 | 29,5 | 24,5 | 20,0 |
| K₂O | 0 | 5 | 5 | 0 | 0 | 0 | 0 | 0 | 0 | 0 | 0 |
| P₂O₅ | 4,0 | 6 | 0 | 15 | 0 | 1 | 7 | 0 | 0 | 0 | 5,0 |
| Ag₂O | 0 | 0 | 0 | 0 | 0 | 0 | 0 | 0,1 | 0,1 | 0 | 0 |
| ZnO | 0 | 0 | 0 | 0 | 0 | 0 | 0 | 0 | 0 | 1,0 | 0 |

Ausf. 2 und 9 neigen schon bei der Schmelze des Glases stark zur Kristallisation. Es ist daher notwendig diese Ausgangsgläser besonders schnell abzukühlen. Falls eine komplette Keramisierung schon bei der Schmelze des Glases auftritt, kann die Glaskeramik einer erneuten Temperung bei den angegebenen Temperaturen unterzogen werden, um die hier beschriebenen Kristallphasen zu erhalten.

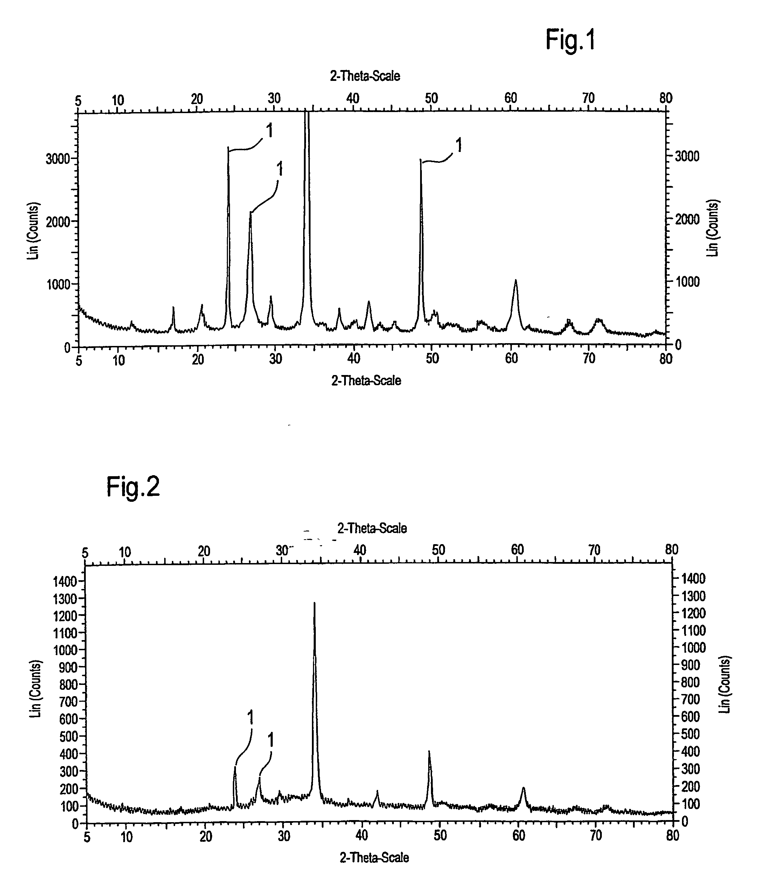

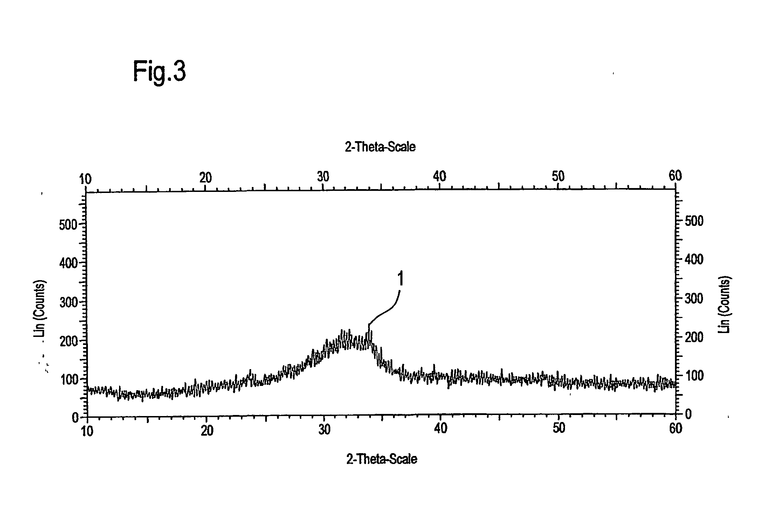

Die Figuren 1 - 3 zeigen die Röntgenbeugungsdiagramme von in Pulverform kristallisierten Ausgangsgläsern gemäß dem Ausführungsbeispiel 1 in Tabelle 1, getempert für 5 h bei 650°C (Figur 1), 590°C (Figur 2) und 560°C (Figur 3). Deutlich zu erkennen die Intensitätsabnahme der auf die Kristallphasen zurückgehenden Beugungsordnungen 1, was gleichbedeutend mit einem sinkenden Kristallanteil in der Glaskeramik ist. Die Intensitätspeaks 1 können beispielsweise Na₂CaSiO₄/Na₂OCaOSiO₂ und Na₂CaSi₃O₈-Kristallphasen zugeordnet werden.

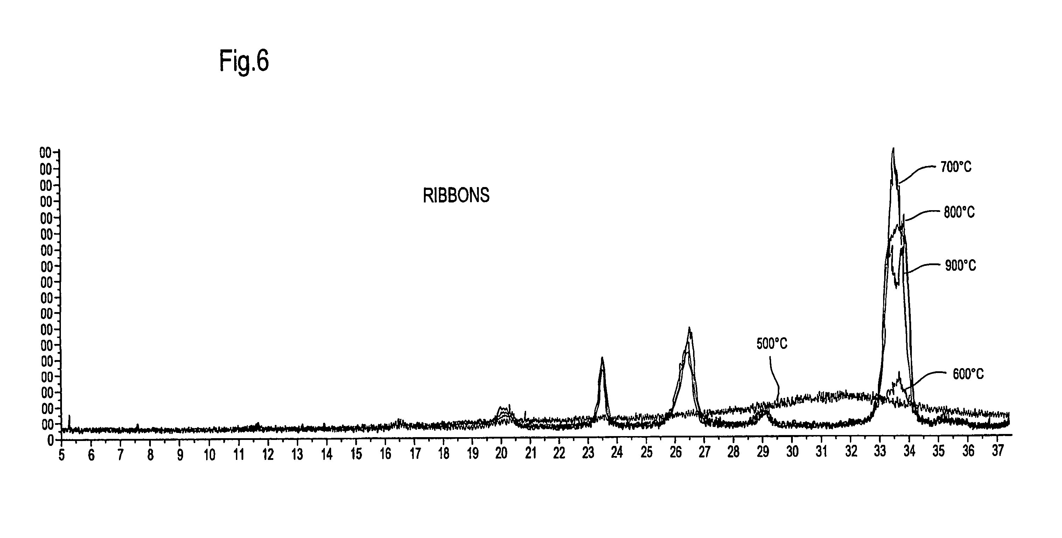

Bei höheren Temperaturen findet eine Umkristallisition statt wie in Figur 6 zu erkennen. Bei Temperaturen > 900°C können sich auch Ca-Silicate bilden.

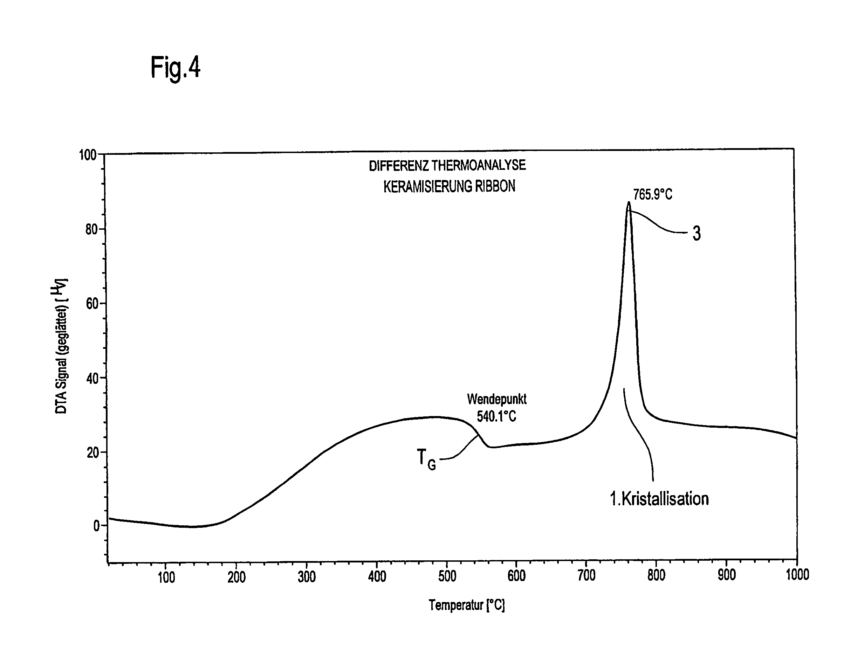

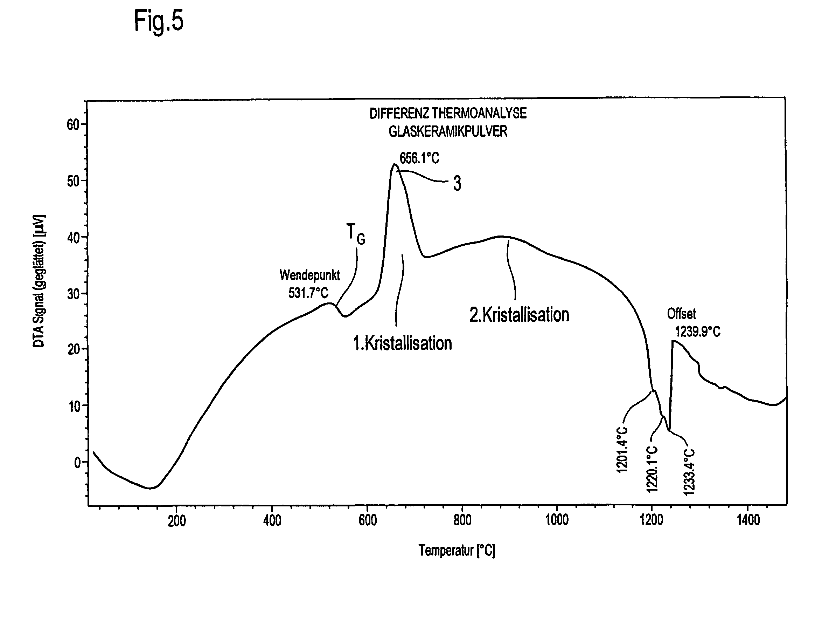

Die Figuren 4 und 5 zeigen die DTA-Thermoanalyse von als Ribbon keramisiertem Ausgangsglas gemäß Ausführungsbeispiel 1 in Tabelle 1 (Figur 4) und in Pulverform keramisierten Ausgangsglas (Figur 5) mit Aufheizraten von 10 K/min. Deutlich zu erkennen der Kristallisationspeak 3 für die Kristallphase, der für das im Pulver keramisierte Ausgangsglas zu geringeren Temperaturen verschoben ist.

In Figur 5 ist auch schwach die exotherme Reaktion der Umkristallisation zu erkennen.

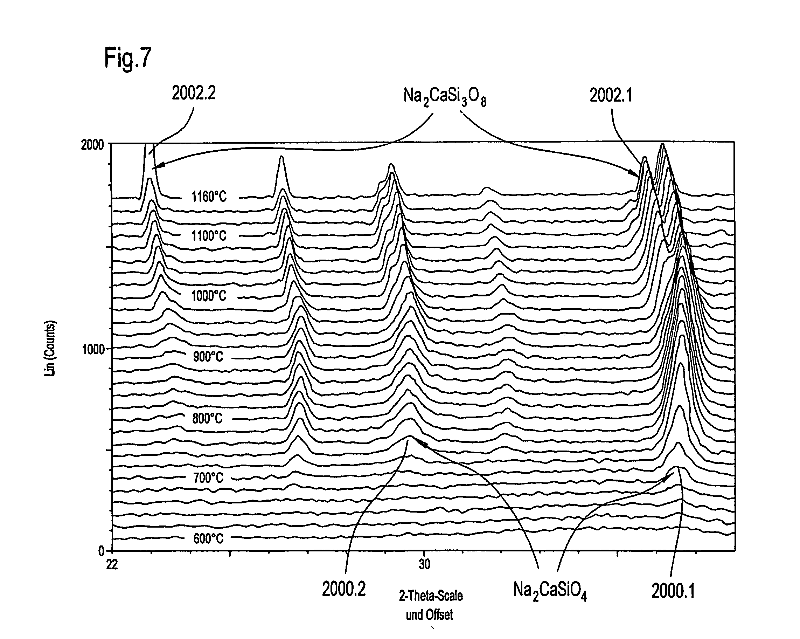

In Figur 7 sind Hochtemperaturröntgendiagramme für ein Glaskeramikpulver, das aus einem Ausgangsglas gemäß Ausführungsbeispiel 7 in Abhängigkeit von der Temperatur erhalten wurde, gezeigt. Die Röntgenmessungen wurden während des Aufheizens aufgenommen.Bei höheren Temperaturen größer 900°C findet eine Umkristallisation statt. Bei diesen Temperaturen können sich auch Ca-Silicate bilden. In Figur 7 ist mit 2000.1 und 2000.2 , die gemäß der JCPDS-Datenbank zuordenbare Na₂CaSiO₄-Phase bezeichnet, mit 2002.1 und 2002.2 die gemäß der JCPDS-Datenbank zuordenbare Na₂CaSi₃O₈-Phase. Wie Figur 7 zu entnehmen wird die Na₂CaSi₃O₈-Phase erst bei Temperaturen oberhalb ca. 900°C gebildet.

Die Eigenschaften der auf verschiedenen Wegen, ausgehend vom Ausgangsglas gemäß Beispiel 1 in Tabelle 1 hergestellten Glaskeramiken ist in Tabelle 2 wiedergegeben.

**Tabelle 2:**

| Eigenschaften von Glaskeramiken gemäß Ausführungsbeispiel 1 | | | | |
|---|---|---|---|---|
| | Temperzeit | Kristalligröße | Kristalline Hauptphasen | JCPDS-Datenbank |
| Pulver 580°C | 5h | < 0,5 | Na₂CaSi₃O₈/ Na₂CaSiO₄ Na₂Ca₂(SiO₃)₃ | 12-0671 /24-10696 |
| Pulver 650°C | 5h | < 1 | Na₂CaSi₃O₈/ Na₂CaSiO₄ Na₂Ca₂(SiO₃)₃ | 12-0671 /24- 10696 |
| Pulver 700°C | 5 h | < 1 | Na₂CaSi₃O₈/ Na₂CaSiO₄ Na₂Ca₂(SiO₃)₃ | 12-0671 / 24-10696 |
| Ribbons 700°C | 5 h | > 100 µm | Na₂CaSi₃O₈/ Na₂CaSiO₄ Na₂Ca₂(SiO₃)₃ | 12-0671 / 24-10696 |
| Ribbons 600°C | 2 h | > 20 µm im Volumen | Na₂CaSi₃O₈/ Na₂CaSiO₄ Na₂Ca₂(SiO₃)₃ | 12-0671 / 24-10696 |

Tabelle 3 zeigt die antibakterielle Wirkung eines Glaskeramikpulvers, das 5 h bei 580°C getempert wurde mit einer Komgröße von 4 µm.

**Tabelle 3:**

| Antibakterielle Wirkung der Pulver nach Europ. Pharmakopoe (3. Auflage): Ausführungsbeispiel 1 (Korngröße 4 µm) | | | | | |
|---|---|---|---|---|---|
| | E.coli | P.aeruginosa | S.aureus | C.albicans | A.niger |
| Start | 290000 | 270000 | 250000 | 300000 | 250000 |
| 2 Tage | 900 | 1800 | 800 | <100 | 2000 |
| 7 Tage | <100 | 200 | <100 | 0 | 2000 |
| 14 Tage | 0 | 0 | 0 | 0 | 0 |
| 21 Tage | 0 | 0 | 0 | 0 | 0 |
| 28 Tage | 0 | 0 | 0 | 0 | 0 |

Bei Hautverträglichkeitstest, d.h. okulsiven Tests über 24 h, wurden keinerlei Hautirritationen festgestellt.
In Tabelle 4 sind in tabellarischer Formbeispielhafte kristallinen Hauptphasen von Na-Ca-Silikatsystemendetailliert angegeben, wobei die allgemeine Formel

x Na₂O · y CaO · z SiO₂

zugrundegelegt wurde und die Zahlen für x, y und z angegeben werden.

**Tabelle 4:**

| Kristalline Hauptphasen von Na-Ca-Silikatsystemen | | |
|---|---|---|
| Na₂O (x) | CaO (y) | SiO₂ (z) |
| 1 | 3 | 6 |
| 1 | 1 | 5 |
| 1 | 2 | 3 |
| 1 | - | 2 |
| 3 | - | 8 |
| 2 | 3 | 6 |
| 2 | - | 2 |
| 0 | 1 | 1 |
| 1 | 0 | 1 |

Nachfolgend sollen die Ergebnisse für Glaskeramiken, die aus Ausgangsgläsern gemäß der Ausführungsbeispiele 8 und 9 erhalten werden, dargestellt werden.

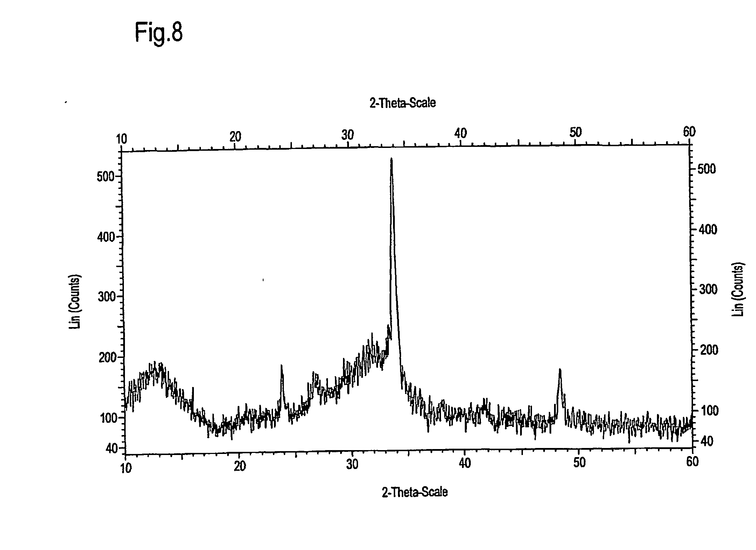

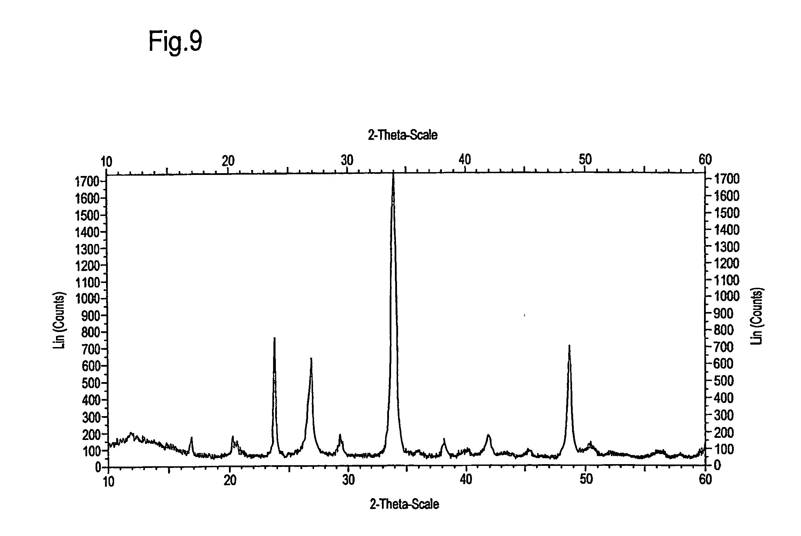

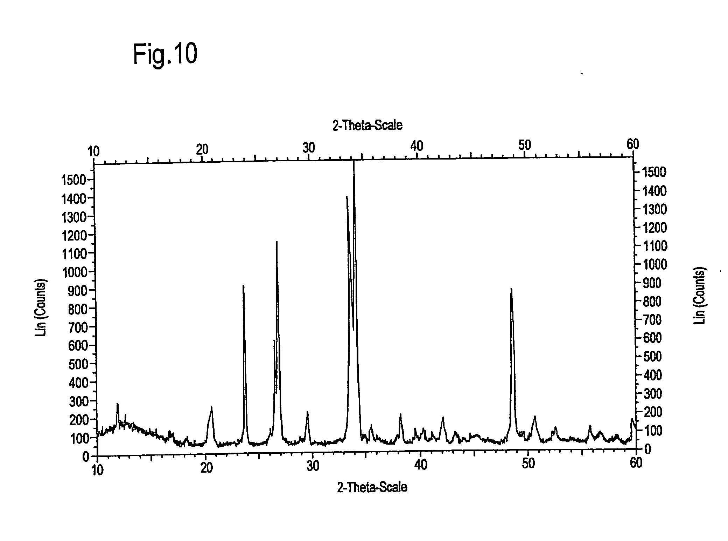

Die Figuren 8 - 10 zeigen die Röntgenbeugungsdiagramme von in Pulverform kristallisierten Ausgangsgläsern gemäß dem Ausführungsbeispiel 8 in Tabelle 1, getempert für 4 Stunden bei 560°C (Figur 8), 700°C (Figur 9) und 900°C (Figur 10). Bei der aus den Intensitätspeaks ermittelbaren Phase handelt es sich um ein Na-Ca-Silikat, und zwar Na₆Ca₃Si₆O₁₈ (JCPDS 77-2189) als kristalline Phase. Deutlich zu erkennen die Änderung des Na-Ca-Verhältnisses mit steigender Temperatur.

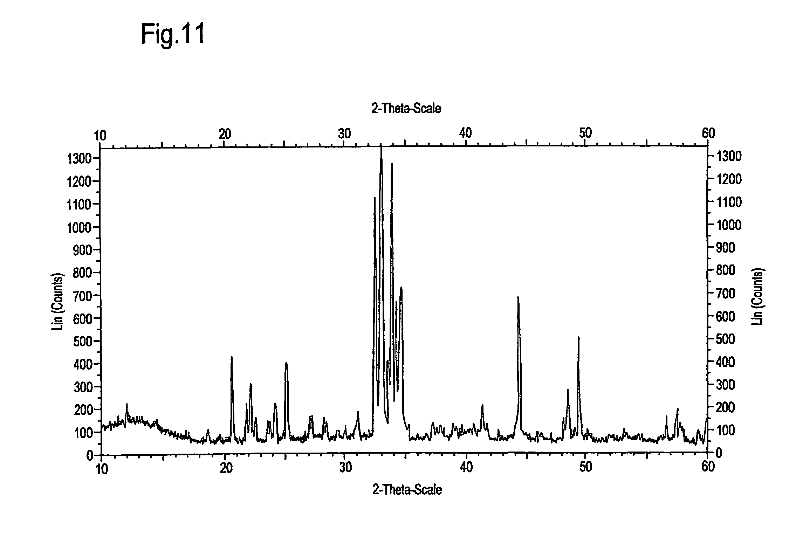

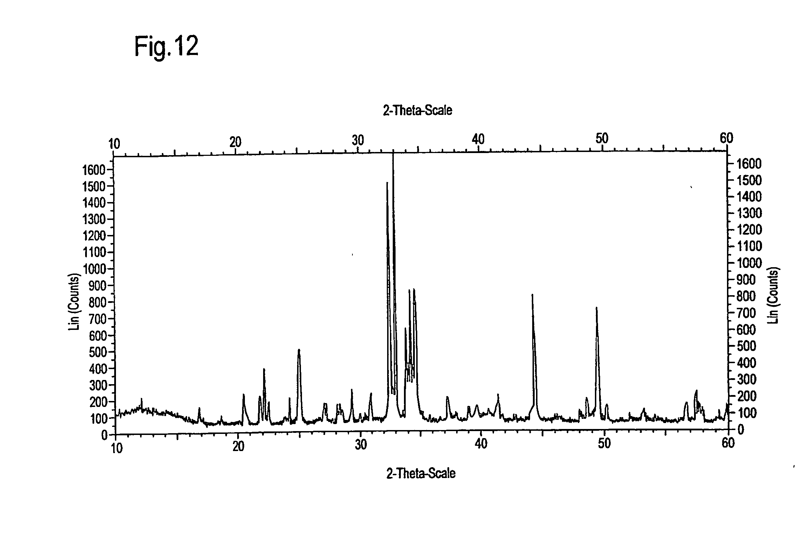

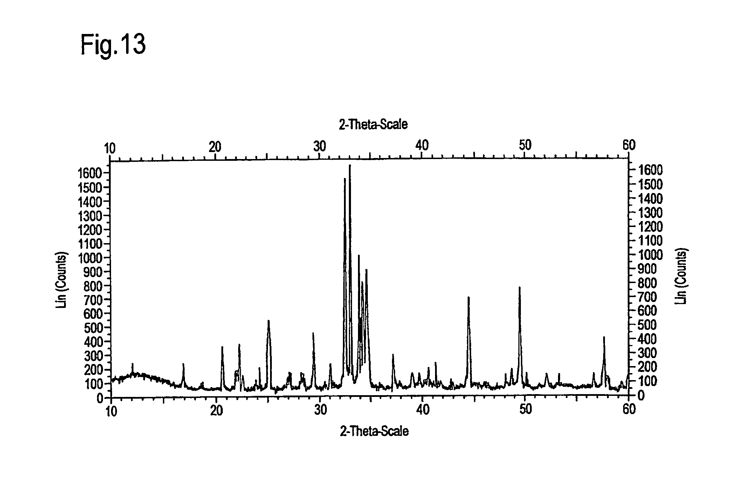

Die Figuren 11 - 13 zeigen die Röntgenbeugungsdiagramme von in Pulverform kristallisierten Ausgangsgläsern gemäß dem Ausführungsbeispiel 9 in Tabelle 1, getempert für 4 Stunden bei 560°C (Figur 11), 700°C (Figur 12) und 900°C (Figur 13).

Als kristalline Hauptphasen können in den Figuren 11 - 13 zwei Na-Ca-Silikate Na₂CaSiO₄ (JCPDS 73-1726) und Na₂Ca₂SiO₇ (JCPDS 10-0016) sowie Siliziumphosphat SiP₂O₇ (JCPDS 39-0189) und Cristobalit SiO₂ (JCPDS 82-0512) identifiziert werden. In den bei 700°C und 900°C hergestellten Proben, die in den Figuren 12 und 13 gezeigt sind, ist eine weitere kristalline Phase enthalten, und zwar Silberphosphat AgPO₃ (JCPDS 11-0641). Der Anteil dieser Phase ist bei der bei 900°C hergestellten Probe größer als bei der bei 700°C hergestellten Probe.

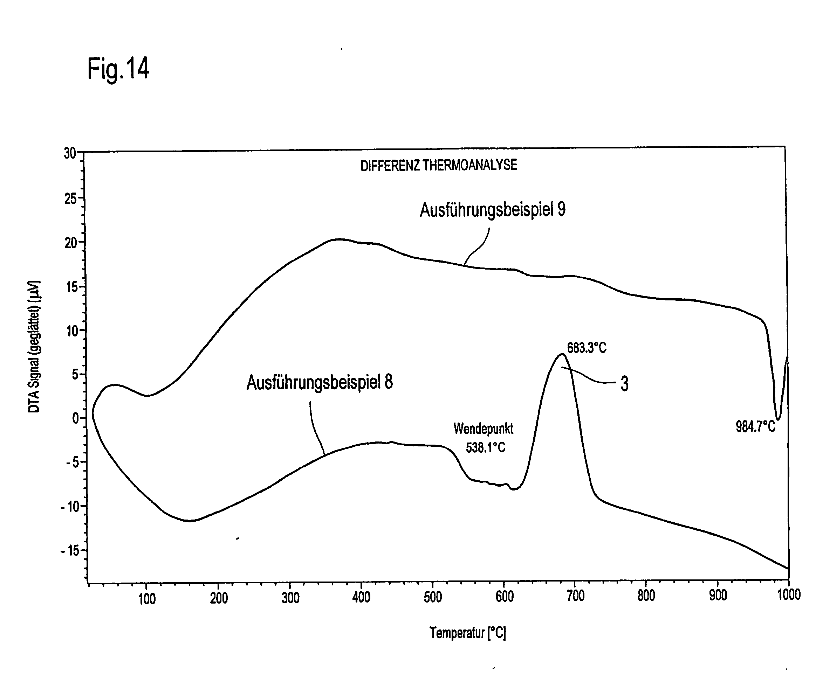

Figur 14 zeigt die DTA-Thermoanalyse von als Ribbon keramisiertem Ausgangsglas gemäß Ausführungsbeispiele 8 und 9 in Tabelle 1 mit Aufheizraten von 10 K/min. Deutlich zu erkennen für das Ausführungsbeispiel 8 ist der Kristallisationspeak 3 für die Kristallphase. Bei der Glaskeramik, die ausgeht vom Ausgangsglas gemäß Ausführungsbeipiel 9 handelt es sich um eine schon aus der Schmelze kristallisierende Glaskeramik. Hierbei ist in der DTA kein stark exothermes Signal mehr zu beobachten, da die Weiter- bzw. Umkristallisation nur noch wenig Wärme freisetzt. Dies ist darauf zurückzuführen, dass das Ausgangsglas in diesem Ausführungsbeispiel bereits beim Erschmelzen zu spontaner Kristallisation neigt.

Tabelle 5 zeigt die antibakterielle Wirkung eines Glaskeramikpulvers, das ausgehend von einem Ausgangsglas gemäß Ausführungsbeispiel 8, bei 560°C. getempert wurde mit einer Komgröße von 4 µm.

**Tabelle 5:**

| Antibakterielle Wirkung der Pulver nach Europ. Pharmakopoe (3. Auflage): Ausführungsbeispiel 8 (Korngröße 4 µm) | | | | | |
|---|---|---|---|---|---|
| | E.coli | P.aeruginosa | S.aureus | C.albicans | A.niger |
| Start | 290000 | 270000 | 250000 | 300000 | 250000 |
| 2 Tage | 700 | 2000 | 500 | <100 | 2000 |
| 7 Tage | 0 | 0 | 0 | 0 | 0 |
| 14 Tage | 0 | 0 | 0 | 0 | 0 |
| 21 Tage | 0 | 0 | 0 | 0 | 0 |
| 28 Tage | 0 | 0 | 0 | 0 | 0 |

Tabelle 6 zeigt zeigt die antibakterielle Wirkung eines Glaskeramikpulvers, das ausgehend von einem Ausgangsglas gemäß Ausführungsbeispiel 9 bei 900° Cgetempert wurde mit einer Komgröße von 4 µm.

**Tabelle 6:**

| Antibakterielle Wirkung der Pulver nach Europ. Pharmakopoe (3. Auflage): Ausf.beisp. 9 (Korngröße 4 µm) | | | | | |
|---|---|---|---|---|---|
| | E.coli | P.aeruginosa | S.aureus | C.albicans | A.niger |
| Start | 290000 | 270000 | 250000 | 300000 | 250000 |
| 2 Tage | 0 | 0 | 0 | 0 | 0 |
| 7 Tage | 0 | 0 | 0 | 0 | 0 |
| 14 Tage | 0 | 0 | 0 | 0 | 0 |
| 21 Tage | 0 | 0 | 0 | 0 | 0 |
| 28 Tage | 0 | 0 | 0 | 0 | 0 |

In Tabelle 7 sind die in den hergestellten Proben aufgefundenen kristallinen Hauptphasen in tabellarischer Form detailliert angegeben, wobei die allgemeine Formel

x Na₂O × y CaO × z SiO₂

zugrundegelegt wurde und die Zahlen für x, y und z angegeben werden.

Neben den Na-Ca-Phasen wird eine Siliziumphospahtphase gefunden. Außerdem wird bei hohen Temperaturen ab 700°C eine Silberphosphat-Phase gefunden.

**Tabelle 7:**

| Kristalline Hauptphasen der Glaskeramiken Ausf. 8 und 9 | | | | | |
|---|---|---|---|---|---|
| Na₂O (x) | CaO (y) | SiO₂ (z) | Ag2O | P2O5 | Bemerkung |
| | | | 1 | 1 | ab 700 |
| 3 | 3 | 6 | | | |
| 1 | 1 | 1 | | | |
| 1 | 2 | 1 | | | |
| | | 1 | | 1 | |
| 2 | 1 | 3 | | | |

In Tabelle 8 werden die pH-Werte und die Leitfähigkeiten einer 1% Suspension eines Glaskeramikpulver, das ein Grundglas gemäß Ausführungsbeispiel 7 in Tabelle 1 umfaßt, für verschiedene Temperbedingungen zur Herstellung der Glaskeramik angegeben. Bei den Temperbedingungen sind die Temperzeiten und die Tempertemperaturen angegeben. Je nach Temperzeit und Tempertemperatur stellen sich verschiedene Hauptkristallphasen in der Glaskeramik ein.

**Tabelle 8:**

| pH-Wert und Leitfähigkeit eines Glaskeramikpulvers | | | | | | |
|---|---|---|---|---|---|---|
| Temperbedingungen | nach 15 min. | | nach 60 min. | | nach 24 Std. | |
| | ph-Wert | Leitfähigkeit (µS/cm) | ph-Wert | Leitfähigkeit (µS/cm) | ph-Wert | Leitfähigkeit (µS/cm) |
| unbehandelt | 11,3 | 695 | 11,3 | 900 | 11,7 | 1672 |
| 500°C - 2h | 11,1 | 526 | 11,2 | 623 | 11,4 | 1054 |
| 600°C - 2h | 11,2 | 571 | 11,2 | 686 | 11,5 | 1130 |
| 700°C - 2h | 11,2 | 576 | 11,2 | 679 | 11,5 | 1007 |
| 800°C - 2h | 11,2 | 619 | 11,3 | 746 | 11,5 | 1138 |
| 900°C - 2h | 11,3 | 859 | 11,4 | 949 | 11,5 | 1288 |

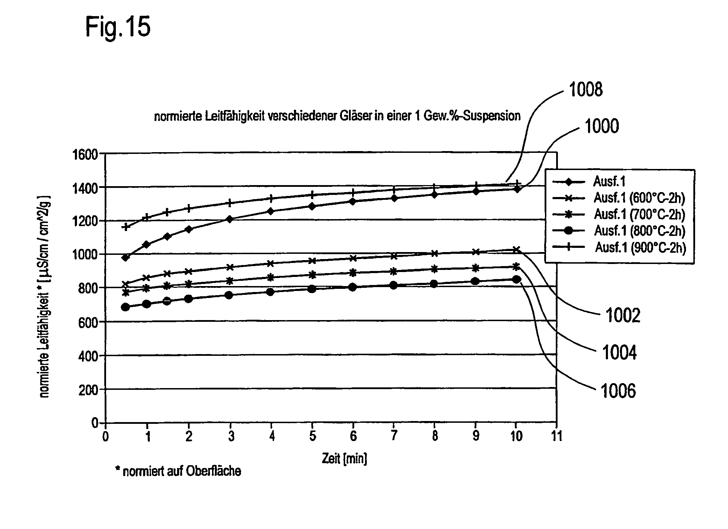

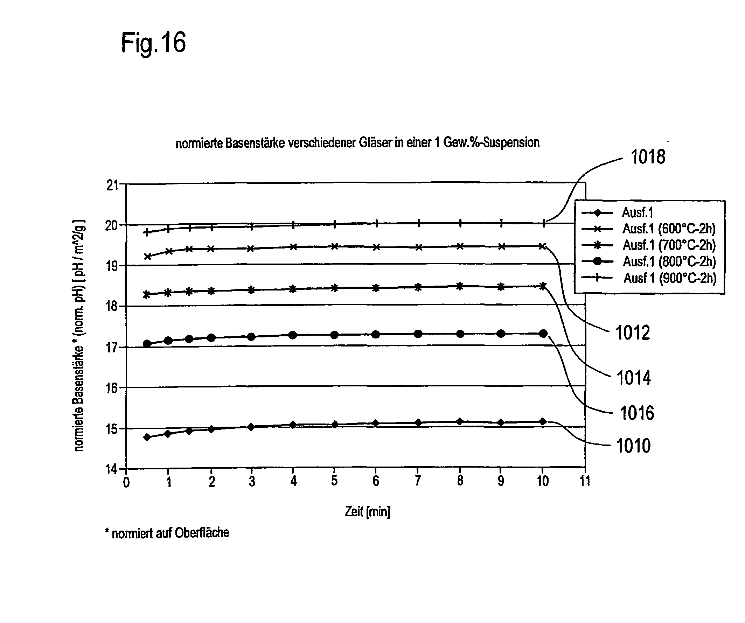

In den Figuren 15 und 16 werden die pH-Werte, d.h. die Basenstärken und die Leitfähigkeiten einer 1% Suspension eines Glaskeramikpulver, das ein Grundglas gemäß Ausführungsbeispiel 1 in Tabelle 1 umfasst, für verschiedene Temperbedingungen zur Herstellung der Glaskeramik angegeben. Die Keramisierungsbedingungen sind für die jeweiligen Glaspulver in Klammern angegeben. Es ist der zeitliche Verlauf dieser Größen gezeigt. Die Normierung bezieht sich auf die spezifische Oberfläche der Partkel (m²/g). Bei den Temperbedingungen sind die Temperzeiten und die Tempertemperaturen angegeben. Je nach Temperzeit und Tempertemperatur stellen sich verschiedene Hauptkristallphasen in der Glaskeramik ein.

In Figur 15 ist der zeitliche Verlauf der normierten Leüfähigkeit für ein Glaskeramikpulver, das ein Grundglas gemäß Ausführungsbeispiel 1 in Tabelle 1 umfasst, gezeigt. Hierbei beschreibt die Kurve 1000 die Leitfähigkeit eines nicht keramisierten Ausgangsglases, 1002 die Leitfähigkeit eines bei 600° C für 2 h getemperten Ausgangsglases, 1004 die Leitfähigkeit eines bei 700° C für 2 h getemperten Ausgangsglases, 1006 die Leitfähigkeit eines bei 800° C für 2 h getemperten Ausgangsglases, 1008 die Leitfähigkeit eines bei 900° C getemperten Ausgangsglases.

Aus Figur 15 ist erkennbar, dass die Leitfähigkeit, d. h. der Anteil der mobilen Ionen in der Lösung, über die Keramisierung hervorragend einstellbar ist.

In Figur 16 ist der zeitliche Verlauf der normierten Basenstärke für ein Glaskeramikpulver, das ein Grundglas gemäß Ausführungsbeispiel 1 in Tabelle 1 umfasst, gezeigt. Hierbei beschreibt die Kurve 1010 die Basenstärke eines nicht keramisierten Ausgangsglases, 1012 die Basenstärke eines bei 600° C für 2 h getemperten Ausgangsglases, 1014 die Basenstärke eines bei 700° C für 2 h getemperten Ausgangsglases, 1016 die Basenstärke eines bei 800° C für 2 h getemperten Ausgangsglases, 1018 die Basenstärke eines bei 900° C getemperten Ausgangsglases.

Aus Figur 16 ist erkennbar, dass die Basizität, d. h. die Einstelllung der Menge gesamt ausgetauschter Ionen, über die Keramisierung hervorragend einstellbar ist. Der auf die spezifische Oberfläche normierte pH-Wert stellt hier ein Maß für die gesamt ausgetauschten Ionen dar.

Aus Figur 16 ist ersichtlich, das z.B. bei der Glaskeramik, die 2h bei 900°C keramisiert wurde, eine deutlich höhere Reaktivität im Vergleich zum unkeramisierten Glas erreicht werden konnte.

Aus der Betrachtung der Figuren 15 und 16, d. h. der norm. pH und norm. Leitfähigkeit ist erkennbar, dass bei der Glaskeramik mit der Bezugsziffer 1008, 1018 normierten Basizität (900°C/2h) im Vergleich zum unkeramisierten Glas eine deutlich erhöhte Mineralbildung, wie z.B. Hydroxylapatit o.ä. auftritt. Die normierten Leitfähigkeiten, d. h. die mobilen Ionen in der Lösung, sind in erster Näherung gleich, die Anzahl der austauschten Ionen ist jedoch bei der Glaskeramik um ein Vielfaches höher. Hier wird auf die logarithmische Darstellung der y-Achse verwiesen. Da die ausgetauschten Ionen nicht mehr mobil in der Lösung sind und somit nicht mehr zur Leitfähigkeit beitragen können, sind sie aus der Lösung ausgefällt.

Eine Quantifizierung der ausgefällten Kristalle mit Analytischen Methoden ist aufgrund der teilweise gebildeten Nanokristalle nur schwer möglich.

In Tabelle 9 ist die lonenlässigkeit von nicht keramisierten Pulver und Glaskeramikpulver in 1% Suspension die als Ausgangsglas ein Glas gemäß Ausführungsbeispiel 7 in Tabelle 1 umfasst gezeigt. Das Glaskeramikpulver wurde durch Tempern bei 650°C für 4 h hergestellt.

**Tabelle 9:**

| lonenlässigkeit (1 %ige Suspension, Einheit mg/Liter) | | | |
|---|---|---|---|
| | nicht keramisiert | Pulver keramisiert 650°C/4h | Pulver keramisiert 900°C/4h |
| Na | 96,7 mg/Liter | 63,2 mg/Liter | 90,7 mg/Liter |
| Ca | 29,9 mg/Liter | 21,5 mg/Liter | 26,8 mg/Liter |
| Si | 63,5 mg/Liter | 40,3 mg/Liter | 59,2 mg/Liter |
| P | 0,22 mg/Liter | 0,67 mg/Liter | 0,19 mg/Liter |

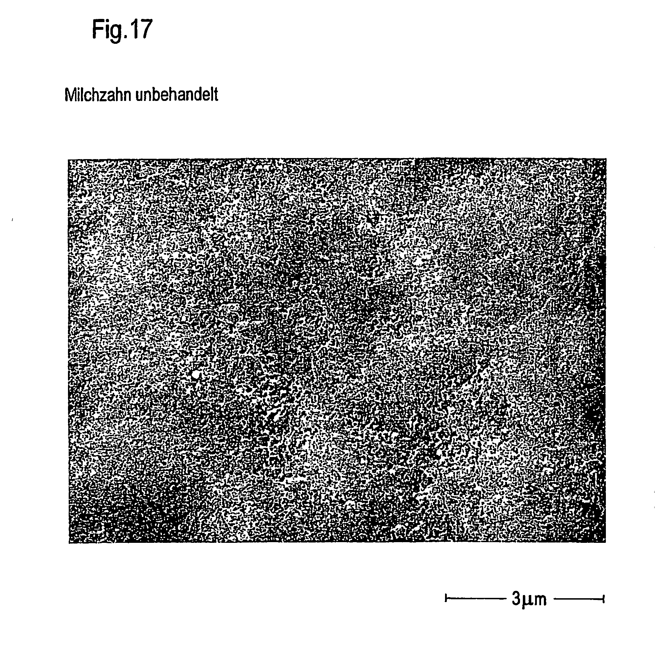

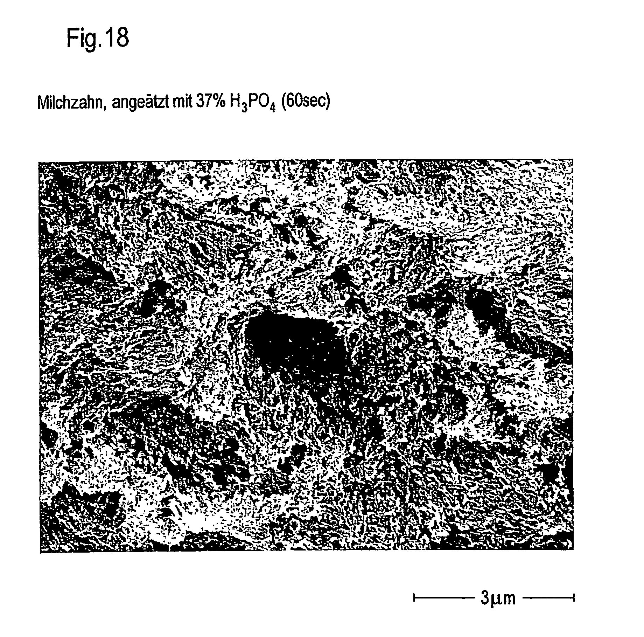

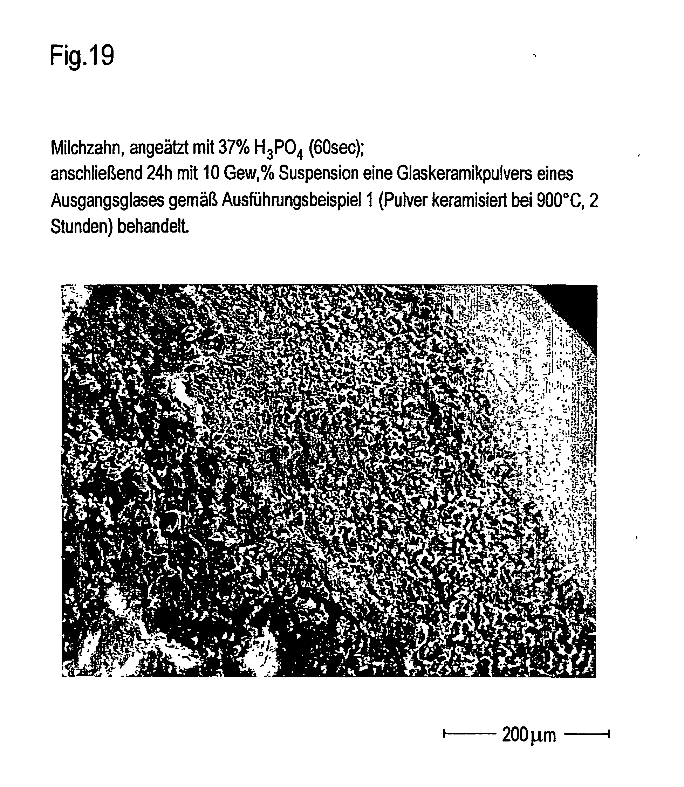

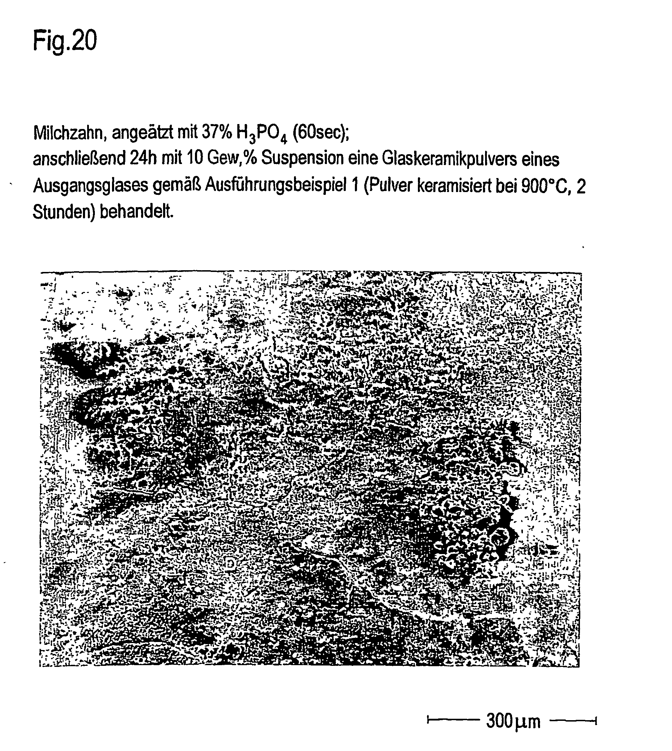

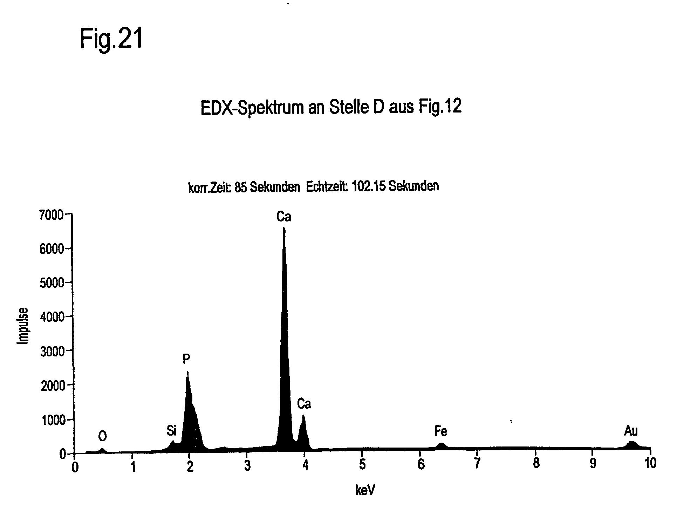

In den Figuren 17 bis 20 sind REM-Aufnahmen eines Milchzahnes gezeigt, und zwar in Figur 17 eines unbehandelten Zahnes, der mit 37%iger Phosphorsäure angelöst wird. Der angelöste Zahn ist in Figur 18 dargestellt. Die Figuren 19 und 20 zeigen den Zahn nach einer Behandlung mit einer 10-Gew% igen wässrigen Suspension eines Glaskeramikpulver in Tris-Puffer. Das Glaskeramikpulver geht aus von einem Ausgangsglas gemäß Ausführungsbeispiel 1. Dieses Ausgangsglas wird bei 900°C für 2 h getempert wurde. Die Partikelgröße beträgt d₅₀=4µm. Der verwandte Tris-Puffer ist: Tris-hydroxyl-methyl-aminomethan. Erkennbar ist sowohl in Figur 19 wie Figur 20 der Aufbau der Mineralschicht. Eine derartige Mineralschicht ist beispielsweise eine Hydroxyl-Apatit-Schicht. Wie aus dem an Position D in Figur 21 aufgenommenen EDX-Spektrum, das in Figur 21 gezeigt ist ,hervorgeht, wird an dieser Stelle eine Ca-Si-P-Schicht, d.h. eine Hydroxy-Apatit-Phase ausgebildet. Der Aufbau einer Mineralschicht hat eine remineralisierende Wirkung für den Zahn.

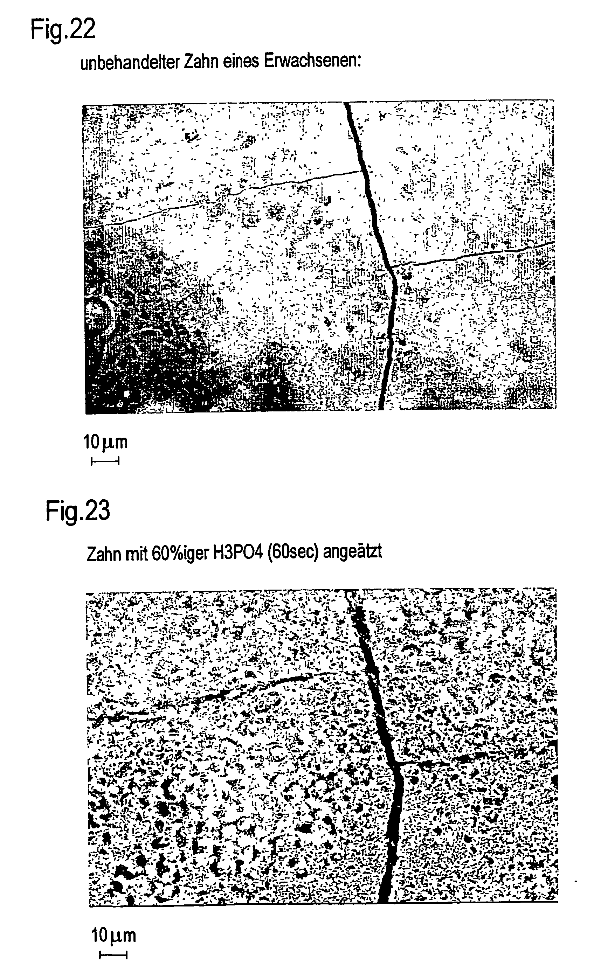

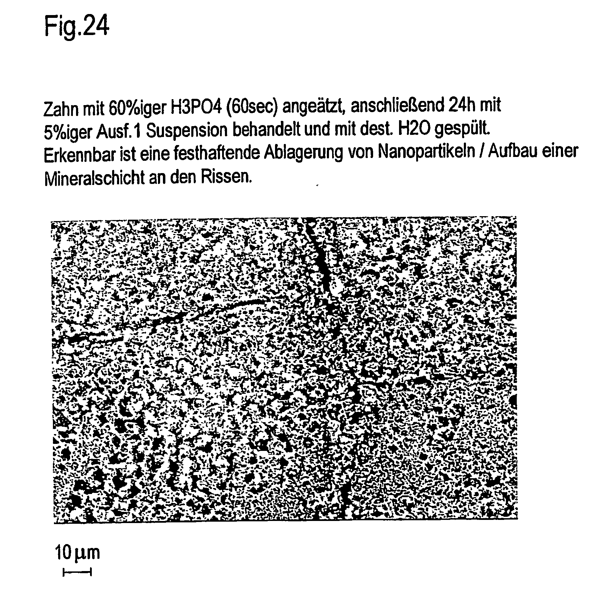

In den Figuren 22 bis 24 sind REM-Aufnahmen eines Zahnes eines Erwachsenen gezeigt, und zwar in Figur 22 eines unbehandelten Zahnes, der mit 60%iger Phosphorsäure angelöst wird. Der für 60 Sekunden angelöste Zahn ist in Figur 23 dargestellt. Die Figur 24 zeigt den Zahn nach einer Behandlung mit einer 5-Gew% igen wässrigen Suspension eines Glaskeramikpulvers.. Das Glaskeramikpulver geht aus von einem Ausgangsglas gemäß Ausführungsbeispiel 1. Dieses Ausgangsglas wird bei 900°C für 2 h getempert wurde. Die Partikelgröße beträgt d₅₀=4µm. Erkennbar ist in Figur 24 der Aufbau der Mineralschicht sowie die festhaftenden Ablagerungen von Nanopartikeln. Eine derartige Mineralschicht ist beispielsweise eine Hydroxyl-Apatit-Schicht. Der Aufbau einer Mineralschicht hat eine remineralisierende Wirkung für den Zahn und wird wie Figur 24 gezeigt an den Rissen ausgebildet.

Nachfolgend sollen Rasterelektronenmikroskopaufnahmen (REM-Aufnahmen) von Glaskeramiken, die durch Kristallisation eines Ausgangsglases gemäß Ausführungsbeispiel 1 erhalten wurden, gezeigt werden.

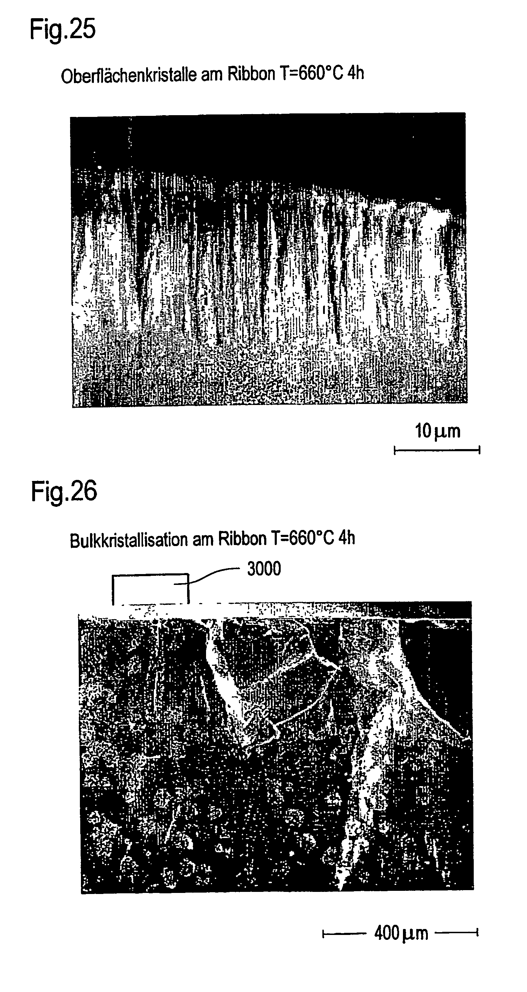

Fig. 25 zeigt eine REM-Aufnahme der Oberfläche einer Glaskeramik, die durch Kristallisation aus einem Ausgangsglas gemäß Ausführungsbeispiel 1 bei einer Temperatur von T=660°C und Tempern für 4h erhalten wurde. Deutlich zu erkennen die Oberflächenkristalle am Ribbon. Teile dieser Oberflächenkristalle können wasserlöslich sein, so dass bei Behandlung mit Wasser diese herausgelöst werden und eine wabenförmige Struktur zurückbleibt. Weiterhin können aus dieser kristallinen Oberfläche bestimmte Phasen als Nanopartikel herausgelöst werden, die unter anderem für Oral Care Anwendungen, d.h. Verwendung der Glaskeramiken der Erfindung im Bereich der Zahn- und Mundpflege wichtig sind. Weiterhin besitzt die in dieser Figur gezeigte kristalline Oberfläche lichtstreuende Eigenschaften, die für bestimmte Anwendungen ausgenutzt werden können.

Während in Figur 25 die Oberflächenstruktur der Glaskeramik gezeigt wurde, ist in Fig.26 eine REM-Aufnahme der Kristallisation im Innern des Glasblocks also die Bulkkristallisation gezeigt. Fig 25 ist ein Ausschnit von Fig 26. Der Ausschnitt ist in Figur 26 mit 3000 bezeichnet.. Die in den Figuren 25 und 26 gezeigte Glaskeramik wurde durch Tempern bei T=660°C für 4h erhalten. Zu erkennen sind die gebildeten Kristallite in Fig. 26 als runde Punkte. Die im Bulk gebildeten Kristalle besitzen lichtstreuende Eigenschaften die für bestimmte Anwendungen ausgenutzt werden können In den Fig 25 und 26 wurde im Glasblock (Ribbon) kristallsiert. Sowohl die Fig 25 wie die Fig 26 zeigen einen Querschnitt durch die Oberfläche des Blockes bzw. Ribbons. Fig 25 ist ein Ausschnitt von Fig 26 und zeigt detailliert die Oberfläche ,

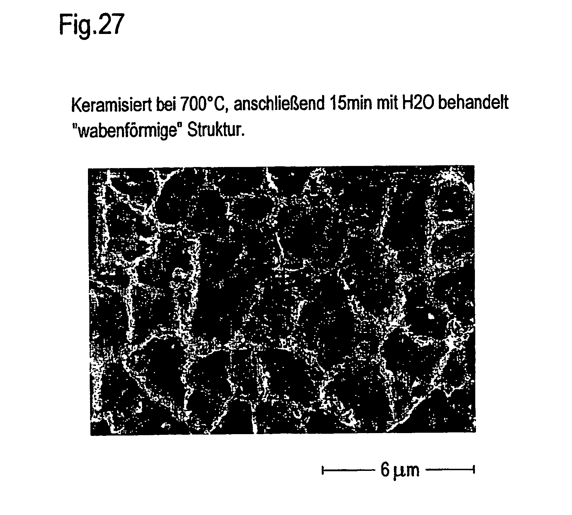

In Figur 27 ist die Oberfläche eines Glaskeramikribbons gezeigt, die durch Keramisierung eines Ausgangsglas gemäß Ausführungsbeispiel 1 durch Tempern bei 700°C für 4 h erhalten wurde. Anschließend wurde die Glaskeramik für 15 min mit H₂O behandelt. Die leicht lösliche Kristallinen Phasen, umfassend im wesentlichen Na-Ca Silikat wird herausgelöst. Es beibt wie in Figur 27 gut zu erkennen eine "wabenförmige" Struktur zurück.

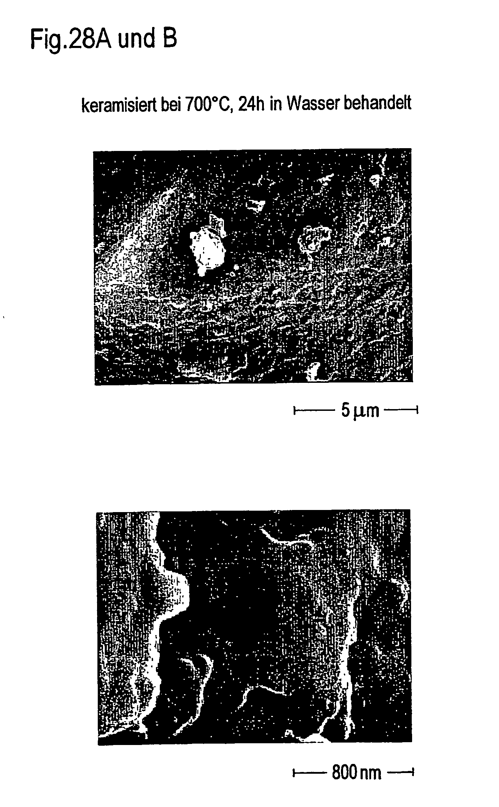

In Figur 28 A und B ist die Oberfläche eines Glaskeramikpulvers gezeigt, das durch Keramisierung eines Ausgangsglases gemäß Ausführungsbeispiel 1 durch Keramisieren bei 700°C für 4 h im Pulver erhalten wurde.. Die gezeigte Oberfläche wurde dadurch erhalten, dass das Glaskeramikpulver für 24h mit Wasser behandelt wurde.
Weiterhin sind in Figur 28A und 28B Oberflächenrauhigkeiten zu erkennen. Wie den Figuren zu entnehmen, ist die Oberfläche relativ homogen und zeigt kaum die Bildung von Nanopartikein.

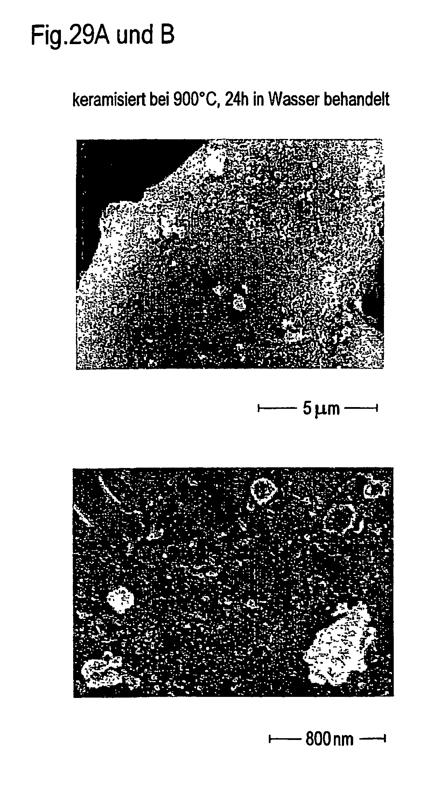

In den Figuren 29A und 29B ist die Oberfläche eines Glaskeramikpulvers gezeigt, das durch Keramisierung eines Ausgangsglases gemäß Ausführungsbeispiel 1 durch Keramisieren bei 900°C für 4 h im Pulver erhalten wurde. Im Gegensatz zu der bei niedrigeren Tempertemperaturen erhaltenen glatten Oberfläche, wie sie in Fig. 28A und 28B gezeigt wurde, sind in den Figuren 29A und 29 herausgelösten Nanokristalle sowie eine poröse Struktur der Oberfläche erkennbar.

Die kristallinen Nanopartikel sind schlechter wasserlöslich. Die Nanopartikel wurden beim Temperschritt gebildet und sind aus der Oberfläche herausgelöst worden.

Die herausgelösten Nanopartikel sind u.a. für Oral Care Anwendungen wichtig, da sie einen desensibilisierenden Effekt auf Zahnnerven besitzen. Die densibilsierende Wirkung wird dadurch erreicht, dass die Nanopartikel die Tubulin-Kanäle verschließen können.

In Tabelle 10 sind die Kristallitgrößen von Glaskeramiken, ausgehend von einem Ausgangsglas gemäß Ausführungsbeispiel 1 für verschiedene Tempertemperaturen angegeben. Bei den Kristallitgrößen handelt es sich um aus der Halbwertsbreite des Röntegnreflexes des Intensitätspeaks 1 des Röntgenbeugungsdiagrammes wie bspw. in den Figuren 1 bis 3 dargestellt ermittelte mittlere Kristallitgröße einer Kristallitgrößenverteilung.

**Tabelle 10:**

| Halbwertsbreite der Glaskeramiken, ermittelt nach der Scherrer-Gleichung mit einem Ausgangsglas gemäß Ausführungsbeispiel 1 für unterschiedliche Tempertemperaturen bei einer Temperzeit von 2h | | | |
|---|---|---|---|
| Tempertemp. | 600°C | 700°C | 900°C |
| Kristallitgröße | 43 nm | 64 nm | 101 nm |

Nachfolgend soll eine Formulierung einer Zahncreme angegeben werden, die 31,06 Gew.-% eines erfindungsgemäßen Glaskeramikpulvers des Ausführungsbeispieles 1 gemäß Tabelle 1 mit 46,0 Gew.-% SiO₂, 25,0 Gew.-% CaO, 25,0 Gew.-% Na₂O und 4 Gew.-% P₂O₅ aufweist:

**Tabelle 10:**

| Zusammensetzung einer Zahncreme mit erfindungsgemäßen Glaskeramikpulver | |
|---|---|
| Komponente | Gew.-% |
| Glycerin (99,3 % rein) | 10,00 |
| Polyethylenglycol 600 | 3,00 |
| Carrageenan (90% aktiv) | 0,85 |
| Natriumsaccarin | 0,40 |
| Natriumfluorid | 0,24 |
| Titandioxid, F.D.&D. Qualität | 0,5 |
| SAPP** (Copolymer von Maleinsäureanhydrid mit Vinylmethylether, durchschnittlich MG ca. 70 000 nach Dampfdruckosomotrie) | 11,5 |
| Wasser*** | 17,8 |
| Sorbitlösung (70%ige wässrige Lösung) | 22,5 |
| Natriumlaurylsulfat | 1,2 |
| Pfefferminzaromastoff**** | 0,95 |
| Glaskeramikpulver mit einem Ausgangsglas einer Grundzusammensetzung gemäß Ausführungsbeispiel 1 in Tabelle 1, keramisiert bei 580°C für 4h, Korngröße bzw. Partikelgröße d₅₀=4µm | 31,06 |

| | |
|---|---|
| *) Viscarin TP-206, hergestellt von Marine Colloids Division of FMC Corporation **) Gantrez S-97, flüssig, hergestellt von CAF Corporation | |
| ***) entmineralisiert und bestrahlt (Behandlung mit UV-Strahlen) | |
| ****) umfasst Menthol | |

Insbesondere eignen sich die erfindungsgemäßen Glaskeramikpulver auch für eine Verwendung in wasserfreien Formulierungen, beispielsweise wasserfreien Zahncremes etc.

Mit der Erfindung werden erstmals ein Glaskeramikpulver und eine Glaskeramik zur Verfügung gestellt, die im Bereich der Zahnpflege und der Mundhygiene in vorteilhafter Weise verwendet werden können. Insbesondere eignet sich die erfindungsgemäße Glaskeramik bzw. das erfindungsgemäße Glaskeramikpulver als Zusatz in Formulierungen für die Mundhygiene bzw. die Zahnpflege, beispielsweise in Zahncremes. Durch das Einbringen von Glaskeramikpulver anstelle von Glaspulvern, beispielsweise in Formulierungen für die Zahnpflege, werden die mechanischen Eigenschaften stark verbessert. Durch Mahlung erfolgt eine tribologische Aktivierung und somit eine höhere Reaktivität der Pulver. Für Dentalanwendungen sind insbesondere Glaskeramikpulver mit Partikelgrößen < 10 µm, bevorzugt < 5 µm, besonders bevorzugt < 2 µm, insbesondere bevorzugt < 1 µm, geeignet. Im Gegensatz zu Glaspulvern, die in Formulierungen für die Zahnpflege zunächst nur über lonenaustausch Ionen abgeben, lösen sich die kristallinen Phasen eines Glaskeramikpulvers in wässrigen Systemen teilweise auf. Zudem werden sekundäre Nanopartikel erzeugt und die Reaktivität ist gegenüber Glaspulvern stark erhöht.

## Patentansprüche

1. Verwendung einer Glaskeramik, insbesondere eines Glaskeramikpulvers, wobei das Ausgangsglas die nachfolgende Zusammensetzung aufweist (in Gew.-% bezogen auf das Oxid):
| | |
|---|---|
| SiO₂ | 30 - 70 Gew.-% |
| Na₂O | 0 - 40 Gew.-% |
| K₂O | 0 - 40 Gew.-% |
| CaO | 5 - 40 Gew.-% |
| MgO | 0 - 40 Gew.-% |
| Al₂O₃ | 0 - 5 Gew.-% |
| P₂O₅ | 0 - 20 Gew.-% |
| B₂O₃ | 0 - 5 Gew.-% |
| ZnO | 0 - 10 Gew.-% |
und
0 bis 30 Gew.-% XF_{y}, wobei X Na, K, Mg, Ca sein kann und y = 1 oder y = 2 wobei die Summe Na₂O + K₂O + CaO > 10 Gew% und < 70 Gew.% im Bereich der Mundhygiene sowie der Zahnpflege.

2. Verwendung einer Glaskeramik, insbesondere eines Glaskeramikpulvers, wobei das Ausgangsglas die nachfolgende Zusammensetzung aufweist (in Gew.% bezogen auf das Oxid):
| | |
|---|---|
| SiO₂ | 30 - 70 Gew.-% |
| Na₂O | 0 - 40 Gew.-% |
| K₂O | 0 - 40 Gew.-% |
| CaO | 5 - 40 Gew.-% |
| MgO | 0 - 40 Gew.-% |
| Al₂O₃ | 0 - 5 Gew.-% |
| P₂O₅ | 1 - 20 Gew.-% |
| B₂O₃ | 0 - 5 Gew.-% |
| ZnO | 0 - 10 Gew.-% |
und
0 bis 30 Gew.-% XF_{y}, wobei X Na, K, Mg, Ca sein kann und y = 1 oder y = 2 im Bereich der Mundhygiene sowie der Zahnpflege.

3. Verwendung von Glaskeramiken, insbesondere eines Glaskeramikpulvers, wobei das Ausgangsglas nachfolgende Zusammensetzung umfasst (in Gew.-% auf Oxidbasis):
| | |
|---|---|
| SiO₂ | 30 - 60 Gew.-% |
| K₂O | 0 - 30 Gew.-% |
| Na₂O | 5 - 30 Gew.-% |
| CaO | 5 - 30 Gew.-% |
| P₂O₅ | 2 - 10 Gew.-% |
| ZnO | 0 - 10 Gew.-% |
und
0 bis 30 Gew.-% XF_{y}, wobei X Na, K, Mg, Ca sein kann und y = 1 oder y = 2 im Bereich der Zahnpflege und/oder Mundhygiene.

4. Verwendung von Glaskeramiken, insbesondere eines Glaskeramikpuvers,wobei das Ausgangsglas die nachfolgende Zusammensetzung aufweist:
| | |
|---|---|
| SiO₂ | 30 - 60 Gew.-% |
| K₂O | 5 - 30 Gew.-% |
| Na₂O | 0 - 30 Gew.-% |
| CaO | 5 - 30 Gew.-% |
| P₂O₅ | 2 - 10 Gew.-% |
| ZnO | 0 - 10 Gew.-% |
und
0 bis 30 Gew.-% XF_{y}, wobei X Na, K, Mg, Ca sein kann und y = 1 oder y = 2 im Bereich der Zahnpflege oder der Mundhygiene.

5. Verwendung von Glaskeramiken, insbesondere eines Glaskeramikpulvers, gemäß einem der Ansprüche 1 bis 4, **dadurch gekennzeichnet, dass** im Ausgangsglas die Summe aus Na und K 5 bis 40 Gew.-% beträgt.

6. Verwendung von Glaskeramiken, insbesondere eines Glaskeramikpulvers, nach einem der Ansprüche 1 bis 5 zur Zahnremineralisierung.

7. Verwendung von Glaskeramiken, insbesondere eines Glaskeramikpulvers, nach einem der Ansprüche 1 bis 5 zur Behandlung von Zahnfleischbluten.

8. Verwendung von Glaskeramiken, insbesondere eines Glaskeramikpulvers, nach einem der Ansprüche 1 bis 5 zur Reduktion von Mundgeruch.

9. Verwendung von Glaskeramiken, insbesondere eines Glaskeramikpulvers, nach einem der Ansprüche 1 bis 5 zur Behandlung von Parodontose.

10. Verwendung von Glaskeramiken, insbesondere eines Glaskeramikpulvers, nach einem der Ansprüche 1 bis 5 zur Behandlung von Karies.

11. Verwendung von Glaskeramiken, insbesondere eines Glaskeramikpulvers, nach einem der Ansprüche 1 bis 5 zur Desensibilisierung von Zähnen.

12. Verwendung von Glaskeramiken, insbesondere eines Glaskeramikpulvers, nach einem der Ansprüche 1 bis 5 zur Behandlung von Entzündungen im Oralbereich.

13. Verwendung von Glaskeramiken, insbesondere eines Glaskeramikpulvers, nach einem der Ansprüche 1 bis 5 zur Verhinderung von Zahnsteinbildung.

14. Verwendung einer Glaskeramik, insbesondere eines Glaskeramikpulvers, nach einem der Ansprüche 1 bis 13, wobei die kristalline Hauptphase der Glaskeramik aus Alkali-Erdalkali-Silikaten und Erdalkalisilikaten besteht.

15. Verwendung einer Glaskeramik, insbesondere eines Glaskeramikpulvers, nach Anspruch 14, wobei die kristalline Hauptphase aus Natrium-Calcium-Silikaten und Calcium-Silikaten besteht.

16. Verwendung einer Glaskeramik, insbesondere eines Glaskeramikpulvers, nach einem der Ansprüche 1 bis 15, wobei deren antimikrobielle Eigenschaften durch Metallionen wie Ag, Au, 1, Cu, Ce mit Gesamtanteilen < 2 Gew.-% synergistisch verstärkt werden.

17. Verwendung eines Glaskeramikpulvers nach einem der Ansprüche 1 bis 16, **dadurch gekennzeichnet, dass** die Glaskeramikpulver aus einem Ausgangsglas durch eine Keramisierung in Pulverform hergestellt werden.

18. Verwendung eines Glaskeramikpulvers nach einem der Ansprüche 1 bis 16, **dadurch gekennzeichnet, dass** die Glaskeramikpulver durch eine Keramisierung von Bulkglas bzw. Ribbon mit anschließender Mahlung hergestellt werden.

19. Verwendung einer Glaskeramik gemäß einem der Ansprüche 1 bis 18, **dadurch gekennzeichnet, dass** die Partikelgröße der Glaskeramik < 100 µm ist, bevorzugt < 20 µm, besonders bevorzugt < 5 µm, insbesondere bevorzugt < 1 µm.

20. Formulierung zur Zahnpflege oder Mundhygiene, umfassend 0,1 - 30 Gew.% einer Glaskeramik oder eines Glaskeramikpulvers, wobei das Ausgangsglas
| | |
|---|---|
| SiO₂ | 30 - 70 Gew.-% |
| Na₂O | 0 - 40 Gew.-% |
| K₂O | 0 - 40 Gew.-% |
| CaO | 5 - 40 Gew.-% |
| MgO | 0 - 40 Gew.-% |
| Al₂O₃ | 0 - 5 Gew.-% |
| P₂O₅ | 1 - 20 Gew.-% |
| B₂O₃ | 0 - 5 Gew.-% |
| ZnO | 0 - 10 Gew.% |
und
0 bis 30 Gew.-% XF_{y}, wobei X Na, K, Mg, Ca sein kann und y = 1 oder y = 2 aufweist.

21. Formulierung für die Zahnpflege und/oder Mundhygiene umfassend 0,1 - 30 Gew.-% einer Glaskeramik oder eines Glaskeramikpulvers, wobei das Ausgangsglas der Glaskeramik bzw. des Glaskeramikpulvers die nachfolgenden Zusammensetzungen aufweist:
| | |
|---|---|
| SiO₂ | 30 - 60 Gew.-% |
| K₂O | 0 - 30 Gew.-% |
| Na₂O | 5 - 30 Gew.-% |
| CaO | 5 - 30 Gew.-% |
| P₂O₅ | 2 - 10 Gew.-% |
| ZnO | 0 - 10 Gew.-% |
und
0 bis 30 Gew.-% XF_{y}, wobei X Na, K, Mg, Ca sein kann und y = 1 oder y=2.

22. Formulierung für die Zahnpflege und/oder Mundhygiene umfassend 0,1- 30 Gew.-% einer Glaskeramik oder eines Glaskeramikpulvers, wobei das Ausgangsglas der Glaskeramik bzw. des Glaskeramikpulvers die nachfolgenden Zusammensetzungen aufweist:
| | |
|---|---|
| SiO₂ | 30 - 60 Gew.-% |
| K₂O | 5 - 30 Gew.-% |
| Na₂O | 0 - 30 Gew.-% |
| CaO | 5 - 30 Gew.-% |
| P₂O₅ | 2 - 10 Gew.-% |
| ZnO | 0 - 10 Gew.-% |
und
0 bis 30 Gew.-% XF_{y}, wobei X Na, K, Mg, Ca sein kann und y = 1 oder y=2.

23. Formulierung gemäß einem der Ansprüche 20 bis 22 zur Verwendung in der Zahnpflege zur Zahnremineralisierung.

24. Formulierung gemäß einem der Ansprüche 20 bis 22 zur Verwendung in der Zahnpflege zur Kariesprophylaxe.

25. Formulierung gemäß einem der Ansprüche 20 bis 22 zur Verwendung in der Zahnpflege zur Desensibilisierung von Zähnen.

26. Formulierung gemäß einem der Ansprüche 20 bis 22 zur Verwendung in der Zahnpflege in Zahnpasten, Mundwasser, Zahnseide und Pflegemittel für dritte Zähne.

## Claims

1. Use of a glass ceramic, in particular of a glass ceramic powder, wherein the starting glass has the following composition (in % (m/m), based on the oxide):
| | |
|---|---|
| SiO₂ | 30 - 70 % (m/m) |
| Na₂O | 0 - 40 % (m/m) |
| K₂O | 0 - 40 % (m/m) |
| CaO | 5 - 40 % (m/m) |
| MgO | 0 - 40 % (m/m) |
| Al₂O₃ | 0 - 5 % (m/m) |
| P₂O₅ | 0 - 20 % (m/m) |
| B₂O₃ | 0 - 5 % (m/m) |
| ZnO | 0 - 10 % (m/m) |
and
0 to 30 % (m/m) XF_{y}, where X can be Na, K, Mg or Ca and y = 1 or y = 2, wherein the sum of Na₂O + K₂O + CaO > 10 % (m/m) and < 70 % (m/m), in the field of oral hygiene and dental care.

2. Use of a glass ceramic, in particular of a glass ceramic powder, wherein the starting glass has the following composition (in % (m/m), based on the oxide):
| | |
|---|---|
| SiO₂ | 30 - 70 % (m/m) |
| Na₂O | 0 - 40 % (m/m) |
| K₂O | 0 - 40 % (m/m) |
| CaO | 5 - 40 % (m/m) |
| MgO | 0 - 40 % (m/m) |
| Al₂O₃ | 0 - 5 % (m/m) |
| P₂O₅ | 1 - 20 % (m/m) |
| B₂O₃ | 0 - 5 % (m/m) |
| ZnO | 0 - 10 % (m/m) |
and
0 to 30 % (m/m) XF_{y}, where X can be Na, K, Mg or Ca and y = 1 or y = 2, in the field of oral hygiene and dental care.

3. Use of glass ceramics, in particular of a glass ceramic powder,
wherein the starting glass has the following composition (in % (m/m), based on the oxide)
| | |
|---|---|
| SiO₂ | 30 - 60 % (m/m) |
| K₂O | 0 - 30 % (m/m) |
| Na₂O | 5 - 30 % (m/m) |
| CaO | 5 - 30 % (m/m) |
| P₂O₅ | 2 - 10 % (m/m) |
| ZnO | 0 - 10 % (m/m) |
and
0 to 30 % (m/m) XF_{y}, where X can be Na, K, Mg or Ca and y = 1 or y = 2, in the field of dental care and/or oral hygiene.

4. Use of glass ceramics, in particular of a glass ceramic powder,
wherein the starting glass has the following composition:
| | |
|---|---|
| SiO₂ | 30 - 60 % (m/m) |
| K₂O | 5 - 30 % (m/m) |
| Na₂O | 0 - 30 % (m/m) |
| CaO | 5 - 30 % (m/m) |
| P₂O₅ | 2 - 10 % (m/m) |
| ZnO | 0 - 10 % (m/m) |
and
0 to 30 % (m/m) XF_{y}, where X can be Na, K, Mg or Ca and y = 1 or y = 2, in the field of dental care or oral hygiene.

5. Use of glass ceramics, in particular of a glass ceramic powder, according to one of Claims 1 to 4, **characterised in that** the sum of Na and K in the starting glass is 5 to 40 % (m/m).

6. Use of glass ceramics, in particular of a glass ceramic powder, according to one of Claims 1 to 5 for tooth remineralisation.

7. Use of glass ceramics, in particular of a glass ceramic powder, according to one of Claims 1 to 5 for the treatment of gingival bleeding.

8. Use of glass ceramics, in particular of a glass ceramic powder, according to one of Claims 1 to 5 for the reduction of mouth odour.

9. Use of glass ceramics, in particular of a glass ceramic powder, according to one of Claims 1 to 5 for the treatment of paradontosis.

10. Use of glass ceramics, in particular of a glass ceramic powder; according to one of Claims 1 to 5 for the treatment of caries.

11. Use of glass ceramics, in particular of a glass ceramic powder, according to one of Claims 1 to 5 for desensitisation of teeth.

12. Use of glass ceramics, in particular of a glass ceramic powder, according to one of Claims 1 to 5 for the treatment of inflammation in the oral region.

13. Use of glass ceramics, in particular of a glass ceramic powder, according to one of Claims 1 to 5 to prevent the formation of tartar.

14. Use of a glass ceramic, in particular of a glass ceramic powder, according to one of Claims 1 to 13, wherein the main crystalline phase of the glass ceramic consists of alkali metal - alkaline earth metal silicates and alkaline earth silicates.

15. Use of a glass ceramic, in particular of a glass ceramic powder, according to Claim 14, wherein the main crystalline phase consists of sodium-calcium silicates and calcium silicates.

16. Use of a glass ceramic, in particular of a glass ceramic powder according to one of Claims 1 to 15, wherein the antimicrobial properties thereof are synergistically intensified by metal ions such as Ag, Au, I, Cu, Ce with total content < 2 % (m/m).

17. Use of a glass ceramic powder according to one of Claims 1 to 16, **characterised in that** the glass ceramic powders are produced from a starting glass by ceramicisation in powder form.

18. Use of a glass ceramic powder according to one of Claims 1 to 16, **characterised in that** the glass ceramic powders are produced by ceramicisation of bulk glass or ribbon with subsequent grinding.

19. Use of a glass ceramic according to one of Claims 1 to 18, **characterised in that** the particle size of the glass ceramic is < 100 µm, preferably < 20 µm, particularly preferentially < 5 µm and especially preferentially < 1 µm.

20. Formulation for dental care or oral hygiene containing 01-30 % (m/m) of a glass ceramic or of a glass ceramic powder, wherein the starting glass contains:
| | |
|---|---|
| SiO₂ | 30 - 70 % (m/m) |
| Na₂O | 0 - 40 % (m/m) |
| K₂O | 0 - 40 % (m/m) |
| CaO | 5 - 40 % (m/m) |
| MgO | 0 - 40 % (m/m) |
| Al₂O₃ | 0 - 5 % (m/m) |
| P₂O₅ | 1 - 20 % (m/m) |
| B₂O₃ | 0 - 5 % (m/m) |
| ZnO | 0 - 10 % (m/m) |
and
0 to 30 % (m/m) XF_{y}, where X can be Na, K, Mg or Ca and y = 1 or y = 2.

21. Formulation for dental care and/or oral hygiene containing 0.1 - 30 % (m/m) of a glass ceramic or of a glass ceramic powder, wherein the starting glass for the glass ceramic or the glass ceramic powder has the following compositions:
| | |
|---|---|
| SiO₂ | 30 - 60 % (m/m) |
| K₂O | 0 - 30 % (m/m) |
| Na₂O | 3 - 30 % (m/m) |
| CaO | 5 - 30 % (m/m) |
| P₂O₅ | 2 - 10 % (m/m) |
| ZnO | 0 - 10 % (m/m) |
and
0 to 30 % (m/m) XF_{y}, where X can be Na, K, Mg or Ca and y = 1 or y = 2.

22. Formulation for dental care and/or oral hygiene containing 0.1 - 30 % (m/m) of a glass ceramic or of a glass ceramic powder, wherein the starting glass for the glass ceramic or the glass ceramic powder has the following compositions:
| | |
|---|---|
| SiO₂ | 30 - 60 % (m/m) |
| K₂O | 5 - 30 % (m/m) |
| Na₂O | 0 - 30 % (m/m) |
| CaO | 5 - 30 % (m/m) |
| P₂O₅ | 2 -10 % (m/m) |
| ZnO | 0 - 10 % (m/m) |
and
0 to 30 % (m/m) XF_{y}, where X can be Na, K, Mg or Ca and y = 1 or y = 2.

23. Formulation according to one of Claims 20 to 22 for use in dental care for tooth remineralisation

24. Formulation according to one of Claims 20 to 22 for use in dental care for caries prophylaxis.

25. Formulation according to one of Claims 20 to 22 for use in dental care for desensitisation of teeth.

26. Formulation according to one of Claims 20 to 22 for use in dental care in toothpastes, mouthwashes, dental floss and care agents for third teeth.

## Revendications

1. Utilisation d'une vitrocéramique, en particulier d'une vitrocéramique en poudre, dont le verre de départ présente la composition suivante (en % en poids sur la base de l'oxyde) :
| | |
|---|---|
| SiO₂ | 30-70 % en poids |
| Na₂O | 0-40 % en poids |
| K₂O | 0-40 % en poids |
| CaO | 5-40 % en poids |
| MgO | 0-40 % en poids |
| Al₂O₃ | 0-5 % en poids |
| P₂O₅ | 0-20 % en poids |
| B₂O₃ | 0-5 % en poids |
| ZnO | 0-10 % en poids |
et
0 à 30 % en poids de XF_{y}, où X peut représenter Na, K, Mg, Ca et y = 1 ou y = 2, la somme Na₂O + K₂O + CaO étant supérieure à 10 % en poids et inférieure à 70 % en poids, dans le domaine de l'hygiène buccale ainsi que des soins dentaires.

2. Utilisation d'une vitrocéramique, en particulier d'une vitrocéramique en poudre, dont le verre de départ présente la composition suivante (en % en poids sur la base de l'oxyde) :
| | |
|---|---|
| SiO₂ | 30-70 % en poids |
| Na₂O | 0-40 % en poids |
| K₂O | 0-40 % en poids |
| CaO | 5-40 % en poids |
| MgO | 0-40 % en poids |
| Al₂O₃ | 0-5 % en poids |
| P₂O₅ | 1-20 % en poids |
| B₂O₃ | 0-5 % en poids |
| ZnO | 0-10 % en poids |
et
0 à 30 % en poids de XF_{y}, où X peut représenter Na, K, Mg, Ca et y = 1 ou y = 2, dans le domaine de l'hygiène buccale ainsi que des soins dentaires.

3. Utilisation de vitrocéramiques, en particulier d'une vitrocéramique en poudre, dont le verre de départ présente la composition suivante (sur la base des oxydes) :
| | |
|---|---|
| SiO₂ | 30-60 % en poids |
| K₂O | 0-30 % en poids |
| Na₂O | 5-30 % en poids |
| CaO | 5-30 % en poids |
| P₂O₅ | 2-10 % en poids |
| ZnO | 0-10 % en poids |
et
0 à 30 % en poids de XF_{y}, où X peut représenter Na, K, Mg, Ca et y = 1 ou y = 2, dans le domaine des soins dentaires et/ou de l'hygiène buccale.

4. Utilisation de vitrocéramiques, en particulier d'une vitrocéramique en poudre, dont le verre de départ présente la composition suivante :
| | |
|---|---|
| SiO₂ | 30-60 % en poids |
| K₂O | 5-30 % en poids |
| Na₂O | 0-30 % en poids |
| CaO | 5-30 % en poids |
| P₂O₅ | 2-10 % en poids |
| ZnO | 0-10 % en poids |
et
0 à 30 % en poids de XF_{y}, où X peut représenter Na, K, Mg, Ca et y = 1 ou y = 2, dans le domaine des soins dentaires ou de l'hygiène buccale.

5. Utilisation de vitrocéramiques, en particulier d'une vitrocéramique en poudre, suivant l'une des revendications 1 à 4, **caractérisée en ce que** la somme de Na et K dans le verre de départ a une valeur de 5 à 40 % en poids.

6. Utilisation de vitrocéramiques, en particulier d'une vitrocéramique en poudre suivant l'une des revendications 1 à 5, pour la reminéralisation de dents.

7. Utilisation de vitrocéramiques, en particulier d'une vitrocéramique en poudre suivant l'une des revendications 1 à 4, pour le traitement du saignement des gencives.

8. Utilisation de vitrocéramiques, en particulier d'une vitrocéramique en poudre suivant l'une des revendications 1 à 5, pour réduire la fétidité de l'haleine.

9. Utilisation de vitrocéramiques, en particulier d'une vitrocéramique en poudre suivant l'une des revendications 1 à 5, pour le traitement de la parodontose.

10. Utilisation de vitrocéramiques, en particulier d'une vitrocéramique en poudre suivant l'une des revendications 1 à 5, pour le traitement de la carie.

11. Utilisation de vitrocéramiques, en particulier d'une vitrocéramique en poudre suivant l'une des revendications 1 à 5, pour la désensibilisation de dents.

12. Utilisation de vitrocéramiques, en particulier d'une vitrocéramique en poudre suivant l'une des revendications 1 à 5, pour le traitement d'inflammations dans la région buccale.

13. Utilisation de vitrocéramiques, en particulier d'une vitrocéramique en poudre suivant l'une des revendications 1 à 5, pour empêcher la formation de tartre.

14. Utilisation de vitrocéramiques, en particulier d'une vitrocéramique en poudre suivant l'une des revendications 1 à 13, dans laquelle la phase cristalline principale de la vitrocéramique est constituée de silicates alcalins-alcalinoterreux et de silicates alcalinoterreux.

15. Utilisation de vitrocéramiques, en particulier d'une vitrocéramique en poudre suivant la revendication 14, dans laquelle la phase cristalline principale est constituée de silicates de sodium-calcium et de silicates de calcium.

16. Utilisation d'une vitrocéramique, en particulier d'une vitrocéramique en poudre suivant l'une des revendications 1 à 15, dans laquelle les propriétés antimicrobiennes de la vitrocéramique sont renforcées de façon synergique par des ions métalliques tels que Ag, Au, I, Cu, Ce, en proportions totales inférieures à 2 % en poids.

17. Utilisation d'une vitrocéramique en poudre suivant l'une des revendications 1 à 16, **caractérisée en ce que** les poudres de vitrocéramique sont produites à partir d'un verre de départ par une céramisation sous forme de poudre.

18. Utilisation d'une vitrocéramique en poudre suivant l'une des revendications 1 à 16, **caractérisée en ce que** les poudres de vitrocéramique sont produites par une céramisation de verre en masse ou ribbon, suivie d'un broyage.

19. Utilisation d'une vitrocéramique suivant l'une des revendications 1 à 18, **caractérisée en ce que** le diamètre des particules de vitrocéramique est inférieur à 100 µm, avantageusement inférieur à 20 µm, mieux encore inférieur à 5 µm, notamment inférieur à 1 µm.

20. Formulation pour soins dentaires ou hygiène buccale, comprenant 0,1 à 30 % en poids d'une vitrocéramique ou d'une poudre de vitrocéramique, dont le verre de départ contient
| | |
|---|---|
| SiO₂ | 30-70 % en poids |
| Na₂O | 0-40 % en poids |
| K₂O | 0-40 % en poids |
| CaO | 5-40 % en poids |
| MgO | 0-40 % en poids |
| Al₂O₃ | 0-5 % en poids |
| P₂O₅ | 1-20 % en poids |
| B₂O₃ | 0-5 % en poids |
| ZnO | 0-10 % en poids |
et
0 à 30 % en poids de XF_{y}, où X peut représenter Na, K, Mg, Ca et y = 1 ou y = 2.

21. Formulation pour les soins dentaires et/ou l'hygiène buccale, comprenant 0,1 à 30 % en poids d'une vitrocéramique ou d'une poudre de vitrocéramique, le verre de départ de la vitrocéramique ou de la poudre de la vitrocéramique présentant les compositions suivantes :
| | |
|---|---|
| SiO₂ | 30-60 % en poids |
| K₂O | 0-30 % en poids |
| Na₂O | 5-30 % en poids |
| CaO | 5-30 % en poids |
| P₂O₅ | 2-10 % en poids |
| ZnO | 0-10 % en poids |
et
0 à 30 % en poids de XF_{y}, où X peut représenter Na, K, Mg, Ca et y = 1 ou y = 2.

22. Formulation pour les soins dentaires et/ou l'hygiène buccale, comprenant 0,1 à 30 % en poids d'une vitrocéramique ou d'une poudre de vitrocéramique, le verre de départ de la vitrocéramique ou de la poudre de vitrocéramique présentant les compositions suivantes :
| | |
|---|---|
| SiO₂ | 30-60 % en poids |
| K₂O | 5-30 % en poids |
| Na₂O | 0-30 % en poids |
| CaO | 5-30 % en poids |
| P₂O₅ | 2-10 % en poids |
| ZnO | 0-10 % en poids |
et
0 à 30 % en poids de XF_{y}, où X peut représenter Na, K, Mg, Ca et y = 1 ou y = 2.

23. Formulation suivant l'une des revendications 20 à 22, destinée à être utilisée dans les soins dentaires pour la reminéralisation de dents.

24. Formulation suivant l'une des revendications 20 à 22, destinée à être utilisée dans les soins dentaires pour la prophylaxie de la carie.

25. Formulation suivant l'une des revendications 20 à 22, destinée à être utilisée dans les soins dentaires pour la désensibilisation de dents.

26. Formulation suivant l'une des revendications 20 à 22, destinée à être utilisée pour les soins dentaires dans des pâtes dentifrices, des collutoires, de la soie dentaire et dans des produits pour les soins de troisièmes dents.
